# EUROPEAN PATENT APPLICATION

(11) **EP 4 653 447 A1**
(43) Date of publication of application: **26.11.2025**
(21) Application number: 24744340.1
(22) Date of filing: 18.01.2024
(51) Int. Cl.: C07H 15/26, C07H 21/02, C07H 21/00, C12N 15/113, A61K 31/713

(54) **MODIFIED NUCLEOSIDE MONOMER AND DOUBLE-STRANDED RIBONUCLEIC ACID**

(30) Priority: 20.01.2023 CN 202310199895; 14.11.2023 CN 202311510663
(71) Applicant: CSPC Zhongqi Pharmaceutical Technology (Shijiazhuang) Co., Ltd., Shijiazhuang, Hebei 050035 (CN)
(72) Inventor: SU, Xiaoye, Shijiazhuang, Hebei 050035 (CN); ZHAO, Chenglong, Shijiazhuang, Hebei 050035 (CN); WANG, Lingyu, Shijiazhuang, Hebei 050035 (CN); ZHANG, Xueyan, Shijiazhuang, Hebei 050035 (CN); CHEN, Huiyu, Shijiazhuang, Hebei 050035 (CN); SHAO, Yu, Shijiazhuang, Hebei 050035 (CN); MA, Chengxin, Shijiazhuang, Hebei 050035 (CN); LI, Chunlei, Shijiazhuang, Hebei 050035 (CN)
(74) Representative: Gille Hrabal Partnerschaftsgesellschaft mbB Patentanwälte
(86) International application number: PCT/CN2024/072943
(87) International publication number: WO 2024/153166

(57) **Abstract**

The present disclosure relates to the field of biomedicine, and in particular to a modified nucleoside monomer compound, a double-stranded ribonucleic acid containing same with reduced off-target effects or improved activity of inhibiting gene expression, and use for manufacturing a medicament for inhibiting target gene expression in mammalian cells and a composition comprising the medicament.

## Description

The present application claims priority to the prior application with the patent application No. 202310199895.5 filed with China National Intellectual Property Administration on Jan. 20, 2023 and the prior application with the patent application No. 202311510663.3 filed on Nov. 14, 2023, both of which are entitled "MODIFIED NUCLEOSIDE MONOMER AND DOUBLE-STRANDED RIBONUCLEIC ACID". These two prior applications are herein incorporated by reference in their entirety.

### TECHNICAL FIELD

The present disclosure relates to the field of biomedicine, and in particular to a modified nucleoside monomer compound, a double-stranded ribonucleic acid containing same with reduced off-target effects or improved activity of inhibiting gene expression, and use for manufacturing a medicament for inhibiting target gene expression in mammalian cells and a composition comprising the medicament.

### BACKGROUND

RNA interference or "RNAi" is a term originally proposed by the Fire group to describe the observation that double-stranded RNA (dsRNA) can block gene expression, and short dsRNA directs gene-specific post-transcriptional silencing in many organisms, including vertebrates, which has provided a new tool for the study of gene function (Fire et al., Nature., (1998), 391(6669): 806-811; Elbashir et al., Genes Dev., (2001), 15, 188-200). The endonuclease Dicer in the cytoplasm of the host cell cleaves the dsRNA into a plurality of small RNA fragments (about 21-23 bp) with specific lengths and structures, i.e., siRNA. siRNA is directly involved in the RNAi process. Specifically, RNAi is mediated by an RNA-induced silencing complex (RISC). RISC specifically binds to related mRNA and has the function of a nuclease, cleaving mRNA at the binding site, and then the cleaved mRNA degrades.

RNAi drugs treat protein-related diseases by degrading target mRNA genes and thereby reducing target proteins expressed by target genes. In contrast, antibody drugs treat diseases at the protein level through affinity with target proteins and the like, and RNAi regulates precursors of protein expression at the mRNA level, which is a therapeutic technology further upstream.

According to the disclosure of Nature Reviews Drug Discovery, currently approved drugs worldwide can interact with about 700 proteins (encoded by about 0.05% of the genome). However, among human disease-related pathogenic proteins, about more than 80% of the proteins cannot be targeted by current conventional small-molecule drugs and biomacromolecule formulations and are non-druggable protein targets. RNA-targeted drugs represented by RNAi have the advantages of abundant candidate targets, short research and development period, lasting efficacy, and a high success rate of clinical development. Treatment at the post-transcriptional level thereof can achieve a breakthrough for special protein targets that are difficult to be prepared into drugs, which is expected to overcome diseases without drugs, including genetic diseases and other refractory diseases, and has great potential to develop therapeutic drugs for "untargetable" and "undruggable" diseases. Therefore, it is widely accepted in the industry that: based on the huge therapeutic demand of RNA-targeted drugs, RNA-targeted drugs are expected to become the third wave of modern new drugs following small molecule drugs and antibody drugs.

At present, RNAi-targeted drugs are the mainstream direction of RNA-targeted drugs. Most of the projects are in preclinical or early clinical research, and have great development potential. However, although siRNA has broad prospects for drug development, its extensive clinical application is limited by multiple barriers inside and outside cells. For example, unmodified siRNA has the disadvantages of poor stability, poor pharmacokinetic characteristics, weak gene silencing effect, possible induction of off-target effects, and the like. In addition, the phosphodiester bond of siRNA is vulnerable to RNases and phosphatases. Once in circulation by systemic administration, endonucleases or exonucleases throughout the body will rapidly degrade the siRNA into fragments, preventing the accumulation of intact therapeutic siRNA in the intended tissue.

The off-target effect of siRNA is a key problem that needs to be solved urgently in the research and development of RNAi-targeted drugs, especially the off-target effect caused by a so-called miRNA mechanism. The so-called off-target effect is that when an siRNA drug (i.e., RNAi drug) silences a target gene, due to the argonaute protein binding to the siRNA, the target gene is not silenced through the RNAi effect, but the siRNA is treated as a miRNA (microRNA). (Lam et al., Molecular Therapy Nucleic Acids, (2015), 4, e252). Off-targeting caused by siRNAs results from the base complementarity between the seed region (from positions 2-9 of the 5' end) of the RNAi antisense strand loaded with RISC and mRNAs of one or more non-target genes. Off-target effects in siRNAs have been reported in several studies, which depend on the sequence of the seed region and can influence the expression of various genes, leading to up to 30% of positive results in siRNA-based phenotypic screens.

Therefore, the use of chemical modifications to regulate siRNA design to enhance the gene silencing efficacy of siRNA gene therapy or to minimize or reduce off-target effects of siRNA without compromising the gene silencing efficacy of siRNA gene therapy has become a global focus for research and development.

Chinese Invention Patent Application No. CN107075516A discloses a double-stranded RNA (dsRNA) reagent capable of inhibiting the expression of a target gene. The sense strand of the dsRNA reagent comprises at least one thermally labile nucleotide, and at least one thermally labile nucleotide present at the opposite site to the seed region (positions 2-8) of the antisense strand. However, the structure of the monomer disclosed therein is artificially synthesized, not a chemical structure naturally existing in the human body, which may be physiologically toxic. In addition, as a whole, there are many synthesis steps, the production process is high in cost, the process is complex, the requirement for the professionalization of technicians is high, and the like.

### SUMMARY

Based on various defects of the prior art, the present disclosure adopts simpler chemical synthesis steps and methods (fewer technical steps than those disclosed), and reduces off-target effects by modifying some natural ribonucleoside monomers, particularly by in-depth research on the structure of cytosine ribonucleoside, and by modifying the structure thereof and then incorporating it into dsRNA to improve the specific binding, or to minimize the non-specific binding of siRNA to the target while maintaining the specific binding.

In one aspect, the present disclosure provides a modified nucleoside monomer having the structure of formula (I): 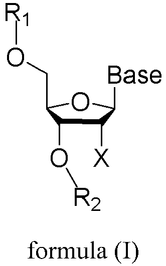 wherein, Base is selected from the following structures: 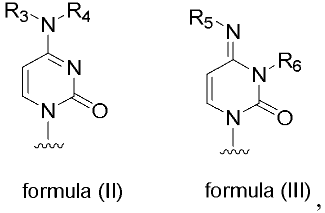
R₃, R₄, R₅, and R₆ are each independently selected from an amino protective group, hydrogen, C₁₋₃ alkyl (methyl, ethyl, propyl, and isopropyl), methoxyethyl, cyclopropyl, fluoromethyl, difluoromethyl, trifluoromethyl, and acetyl, provided that R₃ and R₄ are not both hydrogen, and R₅ and R₆ are not both hydrogen;
X is selected from F and -OMe;
R₁ and R₂ are each independently selected from H, a hydroxyl protecting group, and a phosphoramidite group.

In some embodiments, R₃, R₄, R₅, and R₆ are each independently selected from an amino protective group, hydrogen, C₁₋₃ alkyl (methyl, ethyl, propyl, and isopropyl), and cyclopropyl.

In some embodiments, R₃, R₄, R₅, and R₆ are each independently selected from an amino protective group, hydrogen, methyl, and cyclopropyl.

In some embodiments, R₃ and R₄ are each independently selected from methyl.

In some embodiments, one of R₃ and R₄ is selected from hydrogen, and the other is selected from cyclopropyl.

In some embodiments, Base is selected from

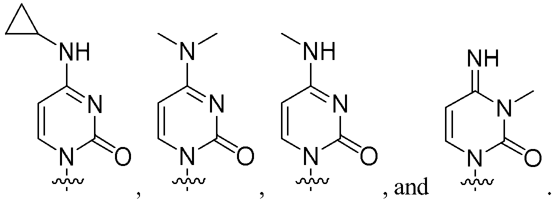

The "amino protective group" described above is selected from benzoyl (Bz), acetyl (Ac), benzyloxycarbonyl (CBz), *tert*-butoxycarbonyl (Boc), 9-fluorenylmethoxycarbonyl (Fmoc), *p-*methoxybenzyl (PMB), benzyl (Bn), trityl (Tr), *p*-toluenesulfonyl (Tos), phthaloyl (Pht), allyloxycarbonyl (Alloc), and trifluoroacetyl (Tfa), preferably, benzoyl (Bz), acetyl (Ac), benzyloxycarbonyl (CBz), *tert*-butoxycarbonyl (Boc), and 9-fluorenylmethoxycarbonyl (Fmoc).

In some embodiments, R₁ and R₂ are each independently selected from a hydroxyl protecting group and a phosphoramidite group.

The "hydroxyl protecting group" described above is selected from methoxymethyl ether (MOM), 2-tetrahydropyran (THP), *tert*-butyldimethylsilyl chloride (TBS), trimethylsilyl (TMS), triethylsilyl (TES), trityl (Tr), 4,4'-dimethoxytrityl (DMTr), 4,4',4'-trimethoxytrityl (TMTr), benzyl (Bn), benzoyl (Bz), acetyl (Ac), and *p*-methoxybenzyl (PMB), preferably trityl (Tr), 4,4'-dimethoxytrityl (DMTr), 4,4',4'-trimethoxytrityl (TMTr), benzyl (Bn), benzoyl (Bz), and acetyl (Ac).

In some embodiments, R₁ and R₂ are each independently selected from DMTr and a phosphoramidite group.

In some embodiments, R₁ is selected from DMTr, and R₂ is selected from a phosphoramidite group.

The "phosphoramidite group" described above is selected from the following structure 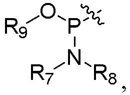 wherein R₇ and R₈ are each independently selected from C₁₋₃ linear or branched alkyl, and R₉ is selected from C₁₋₃ linear or branched alkyl and C₁₋₃ linear or branched alkyl substituted with cyano (-CN).

In some embodiments, R₁ is selected from DMTr, and R₂ is selected from

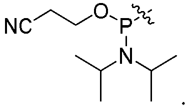

In some embodiments, R₁ and R₂ are both selected from H.

In some embodiments, R₁ is selected from DMTr, and R₂ is selected from H.

In some embodiments, R₁ is selected from H, and R₂ is selected from

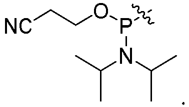

In some embodiments, the modified nucleoside monomer is selected from:

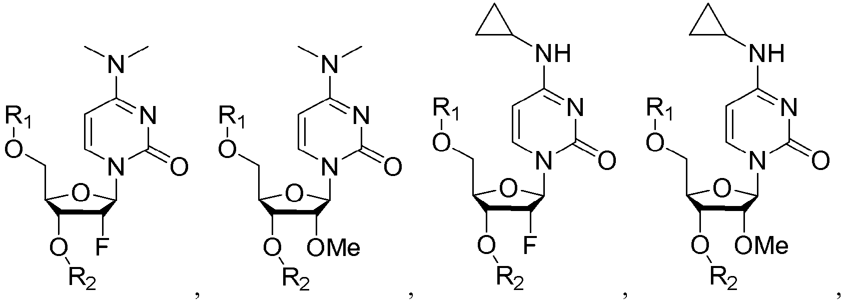

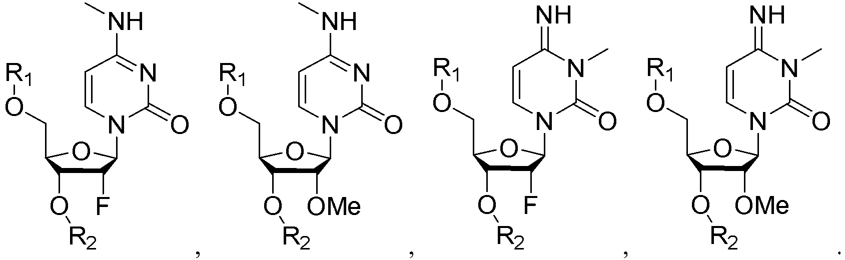

In another aspect, the present disclosure also relates to silencing target gene expression by using double-stranded ribonucleic acid (dsRNA), thereby providing a solution to the problem regarding the treatment of diseases that may be regulated by the down-regulation of the target gene.

The present disclosure provides a double-stranded ribonucleic acid capable of inhibiting target gene expression comprising a sense strand and an antisense strand, wherein each strand has 14-30 nucleotides, the sense strand optionally comprises a ligand, and at least one of positions 6, 7, or 8 from the 5' end of the antisense strand comprises a modified nucleoside monomer as shown below: 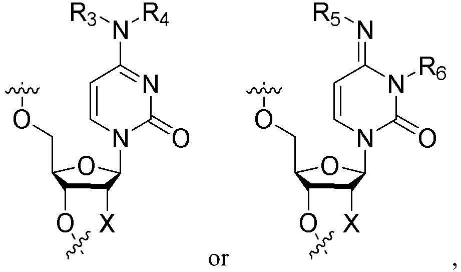 wherein R₃, R₄, R₅, and R₆ are each independently selected from hydrogen, C₁₋₃ alkyl (methyl, ethyl, propyl, and isopropyl), and cyclopropyl, provided that R₃ and R₄ are not both hydrogen, and R₅ and R₆ are not both hydrogen; X is selected from F and -Ome; the wavy line in the structure 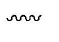 represents a site where a phosphate ester bond is formed.

In some embodiments, at least one of positions 6, 7, or 8 from the 5' end of the antisense strand of the double-stranded ribonucleic acid of the present disclosure comprises a nucleoside monomer as shown below: 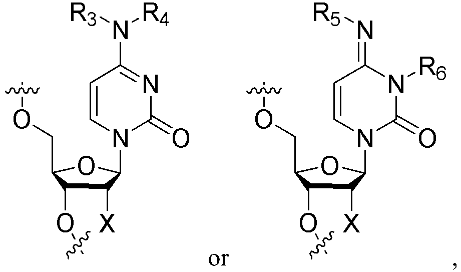 wherein R₃, R₄, R₅, and R₆ are each independently selected from hydrogen, methyl, and cyclopropyl, provided that R₃ and R₄ are not both hydrogen, and R₅ and R₆ are not both hydrogen; X is selected from F and -OMe.

In some embodiments, at least one of positions 6, 7, or 8 from the 5' end of the antisense strand of the double-stranded ribonucleic acid of the present disclosure comprises a nucleoside monomer as shown below: 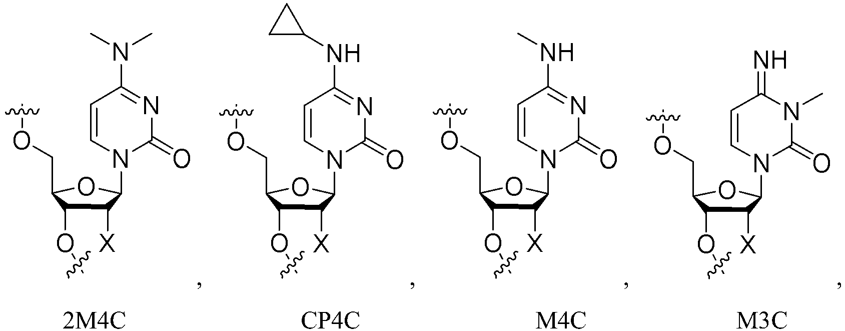 X is selected from F and -OMe.

In some embodiments, the antisense strand of the double-stranded ribonucleic acid of the present disclosure comprises nucleoside monomers 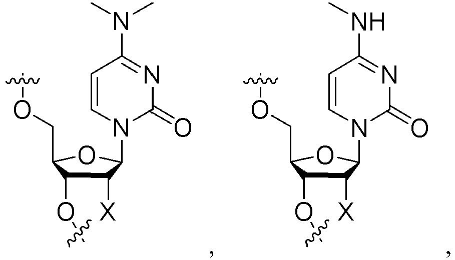 and 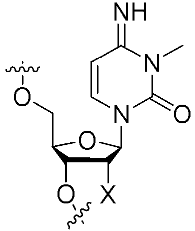 at position 6 from the 5' end.

In some embodiments, the antisense strand of the double-stranded ribonucleic acid of the present disclosure comprises nucleoside monomers 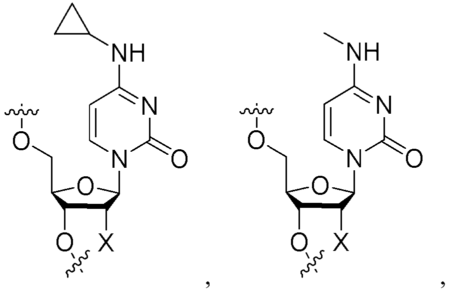 and 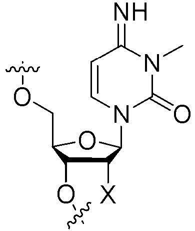 at position 8 from the 5' end.

In some embodiments, the antisense strand of the double-stranded ribonucleic acid of the present disclosure comprises nucleoside monomers 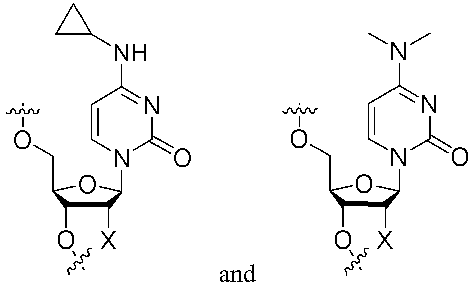 at position 7 from the 5' end.

In some embodiments, at least one of positions 6, 7, or 8 from the 5' end of the antisense strand of the double-stranded ribonucleic acid of the present disclosure comprises the nucleoside monomer

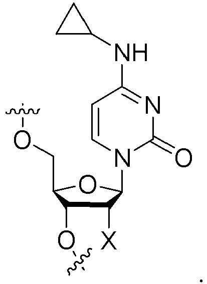

In some embodiments, at least one of positions 7 or 8 from the 5' end of the antisense strand of the double-stranded ribonucleic acid of the present disclosure comprises the nucleoside monomer

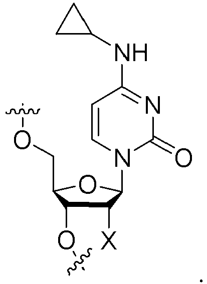

In some embodiments, at least one of positions 6 or 8 from the 5' end of the antisense strand of the double-stranded ribonucleic acid of the present disclosure comprises the nucleoside monomers

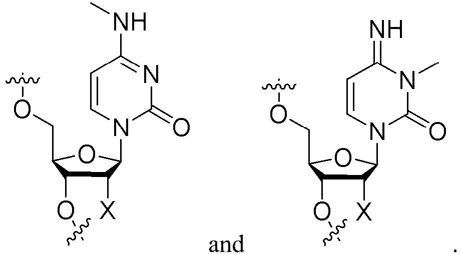

In some embodiments, at least one of positions 6 or 7 from the 5' end of the antisense strand of the double-stranded ribonucleic acid of the present disclosure comprises the nucleoside monomer

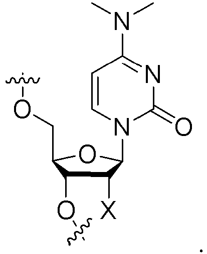

In some embodiments, the antisense strand of the double-stranded ribonucleic acid of the present disclosure comprises the nucleoside monomer 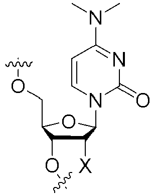 at position 6 from the 5' end.

In some embodiments, the sense strand of the double-stranded ribonucleic acid of the present disclosure comprises a ligand.

In some embodiments, the ligand of the double-stranded ribonucleic acid of the present disclosure is linked at the 5' end or the 3' end of the sense strand.

In some embodiments, the ligand of the double-stranded ribonucleic acid of the present disclosure is an asialoglycoprotein receptor (ASGPR) ligand.

In some embodiments, the ASGPR ligand of the double-stranded ribonucleic acid of the present disclosure is one or more GalNAc derivatives linked through a bivalent or trivalent branched structure.

In some embodiments, examples of one or more GalNAc derivatives linked through a bivalent or trivalent branched structure of the present disclosure include, but are not limited to, the following structures:

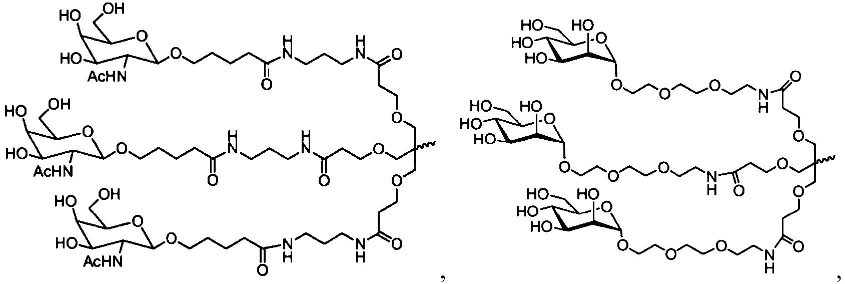

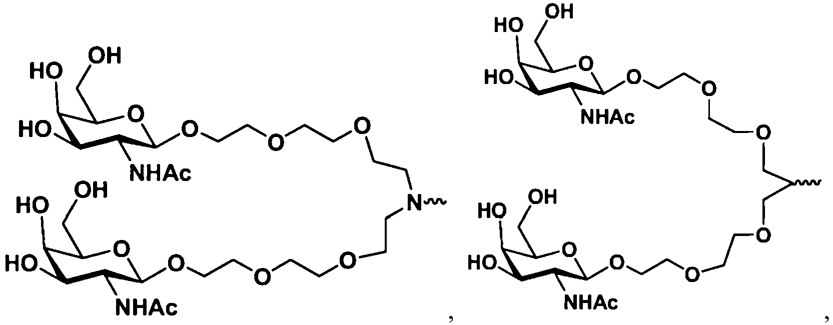

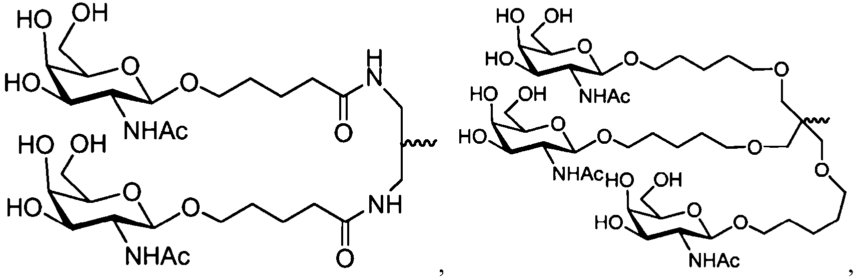

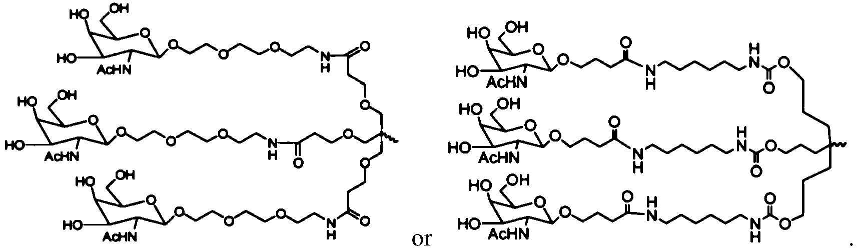

In some embodiments, the sense strand of the double-stranded ribonucleic acid of the present disclosure has 21 nucleotides and the antisense strand has 23 nucleotides.

In another aspect, the present disclosure provides a pharmaceutical composition comprising the double-stranded ribonucleic acid described above and a pharmaceutically acceptable carrier, excipient or delivery carrier.

In some embodiments, the pharmaceutical composition may be delivered via a liposome (e.g., liposome 2000), a virus vector, an extracellular vesicle (EV), a lipid nanoparticle (LNP), or an ASGPR ligand (e.g., one or more GalNAc derivatives) at the 5'-end or 3'-end of the sense strand or antisense strand.

In some embodiments, the virus vector includes, but is not limited to: adenovirus-associated virus (AAV), lentivirus, adenovirus, and retrovirus.

In some embodiments, the pharmaceutical composition may be administered by the following routes or forms: (1) oral administration (aqueous or non-aqueous solutions or suspensions), tablets (e.g., tablets targeted for buccal, sublingual, and systemic absorption), boluses, powders, granules, and pastes for application to the tongue; (2) parenteral administration, e.g., by subcutaneous, intramuscular, intravenous, or epidural injection, preferably delivered by subcutaneous or intravenous methods.

In another aspect, the present disclosure further relates to use of the double-stranded ribonucleic acid or the pharmaceutical composition described above for manufacturing a medicament for inhibiting or silencing target gene expression in a cell.

In some embodiments, the present disclosure relates to use of the double-stranded ribonucleic acid or the pharmaceutical composition described above for manufacturing a medicament for arresting off-target effects caused by the antisense strand of the double-stranded ribonucleic acid.

In some embodiments, the present disclosure relates to use of the double-stranded ribonucleic acid or the pharmaceutical composition described above for manufacturing a medicament for delivering the double-stranded ribonucleic acid to a specific target in a subject.

In another aspect, the present disclosure further relates to a method for inhibiting target gene expression in a cell, comprising the following steps:
(a) introducing the double-stranded ribonucleic acid described above into a cell, wherein the double-stranded ribonucleic acid can inhibit target gene expression by at least 40% when in contact with a cell expressing the target gene; and
(b) maintaining the cell obtained in step (a) for a sufficiently long time to degrade a mRNA transcript of the target gene, thereby inhibiting the expression of the target gene in the cell.

In another aspect, the present disclosure further relates to a method for treating and preventing a disease related to abnormal up-regulation of target gene expression, comprising administering to a patient in need of such treatment, prevention, or control a therapeutically effective amount or a prophylactically effective amount of one or more of the double-stranded ribonucleic acids described above.

In another aspect, the present disclosure further relates to use of the double-stranded ribonucleic acid for manufacturing an inhibitor for target gene expression.

In some embodiments, the target gene is selected from: AGT, ANGPTL3, APOB, BRAF, CTNNB1, C5, CD274, FLT1, FLT2, KIF11, FXII, LPA, PCSK9, and RET, preferably ANGPTL3 and PCSK9 genes, and most preferably PCSK9 gene.

In another aspect, the present disclosure further relates to a method for inhibiting target gene expression in a cell, comprising the following steps:
(a) introducing the double-stranded ribonucleic acid into a cell, wherein the double-stranded ribonucleic acid can inhibit target gene expression by at least 40% when in contact with a cell expressing the target gene;
and (b) maintaining the cell obtained in step (a) for a sufficiently long time to degrade a mRNA transcript of the target gene, thereby inhibiting the expression of the target gene in the cell.

In another aspect, the present disclosure further relates to a method for inhibiting target gene expression in a cell, comprising the following steps:
(a) introducing the double-stranded ribonucleic acid into a cell, wherein the double-stranded ribonucleic acid can inhibit target gene expression by at least 40% when in contact with a cell expressing PCSK9;
and (b) maintaining the cell obtained in step (a) for a sufficiently long time to degrade a mRNA transcript of the target gene, thereby inhibiting the expression of the target gene in the cell.

In some embodiments, the present disclosure further relates to a vector for inhibiting target gene expression in a cell, comprising a regulatory sequence operably linked to a nucleotide sequence encoding at least one strand of the double-stranded ribonucleic acid.

In some embodiments, the present disclosure further relates to a cell comprising the vector for inhibiting target gene expression in a cell. The cell is typically a mammalian cell, e.g., a human cell. In some embodiments, the present disclosure further relates to a pharmaceutical composition for inhibiting target gene expression, which comprises one or more of the double-stranded ribonucleic acids and a pharmaceutically acceptable carrier or delivery carrier.

In some embodiments, the present disclosure further relates to a method for treating and preventing a disease related to abnormal up-regulation of target gene expression, comprising administering to a patient in need of such treatment, prevention, or control a therapeutically effective amount or a prophylactically effective amount of one or more of the double-stranded ribonucleic acids.

In some embodiments, the present disclosure provides a double-stranded ribonucleic acid (dsRNA) molecule for inhibiting the expression of a target gene (e.g., PCSK9). The dsRNA comprises at least two sequences that are complementary to each other. The dsRNA comprises a sense strand having a first sequence and an antisense strand having a second sequence. The antisense strand comprises a nucleotide sequence that is substantially complementary to at least a portion of an mRNA encoded by the target gene, and the region of complementarity is less than 30 nucleotides in length, typically 19-24 nucleotides in length. The dsRNA can inhibit target gene expression by at least 30%, 40%, 50%, 60%, 70%, 80%, and 90% when in contact with a cell expressing the target gene.

In some embodiments, the target gene is selected from: AGT, ANGPTL3, APOB, BRAF, CTNNB1, C5, CD274, FLT1, FLT2, KIF11, FXII, LPA, PCSK9, and RET; preferably ANGPTL3, PCSK9, and LPA, further preferably ANGPTL3 and PCSK9 genes, and most preferably PCSK9 gene.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-AAUGACUUUUAUUGAGCUCUU-3' (SEQ ID NO. 1); and an antisense strand consisting of a nucleotide sequence having at least 90%, preferably 95%, 96%, 97%, 98%, 99%, and 100% identity to the nucleotide sequence 5'-AAGAG/**X**/UCAAUAAAAGUCAUUCU-3' (SEQ ID NO. 2); X is any of the modified nucleoside monomers described above; optionally, the sugar ring and phosphate ester bond of the double-stranded ribonucleic acid may be subjected to conventional chemical modifications to enhance stability.

In other embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-AAUGACUUUUAUUGAGCUCUU-3' (SEQ ID NO. 1); and an antisense strand consisting of a nucleotide sequence having at least 90%, preferably 95%, 96%, 97%, 98%, 99%, and 100% identity to the nucleotide sequence 5'-AAGAGCU/**X**/AAUAAAAGUCAUUCU-3' (SEQ ID NO. 3); **X** is any of the modified nucleoside monomers described above; optionally, the sugar ring and phosphate ester bond of the double-stranded ribonucleic acid may be subjected to conventional chemical modifications to enhance stability.

In some embodiments, X is selected from: M3C, 2M4C, M4C, and CP4C.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCACAACGCUUUUGGGGGUGA-3' (SEQ ID NO. 4); and an antisense strand consisting of a nucleotide sequence having at least 90%, preferably 95%, 96%, 97%, 98%, 99%, and 100% identity to the nucleotide sequence 5'-UCACC/**X**/CCAAAAGCGUUGUGGGC-3' (SEQ ID NO. 5); **X** is any of the modified nucleoside monomers described above; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCACAACGCUUUUGGGGGUGA-3' (SEQ ID NO. 4); and an antisense strand consisting of a nucleotide sequence having at least 90%, preferably 95%, 96%, 97%, 98%, 99%, and 100% identity to the nucleotide sequence 5'-UCACCC/**X**/CAAAAGCGUUGUGGGC-3' (SEQ ID NO. 6); **X** is any of the modified nucleoside monomers described above; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCACAACGCUUUUGGGGGUGA-3' (SEQ ID NO. 4); and an antisense strand consisting of a nucleotide sequence having at least 90%, preferably 95%, 96%, 97%, 98%, 99%, and 100% identity to the nucleotide sequence 5'-UCACCCC/**X**/AAAAGCGUUGUGGGC-3' (SEQ ID NO. 7); X is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-GAGAAUUUUGGUUGGGCCUAG-3' (SEQ ID NO. 8); and an antisense strand consisting of a nucleotide sequence having at least 90%, preferably 95%, 96%, 97%, 98%, 99%, and 100% identity to the nucleotide sequence 5'-CUAGG/**X**/CCAACCAAAAUUCUCCA-3' (SEQ ID NO. 9); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-GAGAAUUUUGGUUGGGCCUAG-3' (SEQ ID NO. 8); and an antisense strand consisting of a nucleotide sequence having at least 90%, preferably 95%, 96%, 97%, 98%, 99%, and 100% identity to the nucleotide sequence 5'-CUAGGC/**X**/CAACCAAAAUUCUCCA-3' (SEQ ID NO. 10); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-GAGAAUUUUGGUUGGGCCUAG-3' (SEQ ID NO. 8); and an antisense strand consisting of a nucleotide sequence having at least 90%, preferably 95%, 96%, 97%, 98%, 99%, and 100% identity to the nucleotide sequence 5'-CUAGGCC/**X**/AACCAAAAUUCUCCA-3' (SEQ ID NO. 11); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCCAGUGUCAGGUGGGAGUAC-3' (SEQ ID NO. 12); and an antisense strand consisting of a nucleotide sequence having at least 90%, preferably 95%, 96%, 97%, 98%, 99%, and 100% identity to the nucleotide sequence 5'-GUACU/**X**/CCACCUGACACUGGGAU-3' (SEQ ID NO. 13); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCCAGUGUCAGGUGGGAGUAC-3' (SEQ ID NO. 12); and an antisense strand consisting of a nucleotide sequence having at least 90%, preferably 95%, 96%, 97%, 98%, 99%, and 100% identity to the nucleotide sequence 5'-GUACUC/**X**/CACCUGACACUGGGAU-3' (SEQ ID NO. 14); **X** is any of the modified nucleoside monomers described above; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-CCCAGUGUCAGGUGGGAGUAC-3' (SEQ ID NO. 12); and an antisense strand consisting of a nucleotide sequence having at least 90%, preferably 95%, 96%, 97%, 98%, 99%, and 100% identity to the nucleotide sequence 5'-GUACUCC/**X**/ACCUGACACUGGGAU-3' (SEQ ID NO. 15); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-GUGAUUCAAGUUUAUGGAUAC-3' (SEQ ID NO. 16); and an antisense strand consisting of a nucleotide sequence having at least 90%, preferably 95%, 96%, 97%, 98%, 99%, and 100% identity to the nucleotide sequence 5'-GUAUC/X/AUAAACUUGAAUCACAA-3' (SEQ ID NO. 17); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-UGUGUAUUUGGAAGUUGUAUC-3' (SEQ ID NO. 18); and an antisense strand consisting of a nucleotide sequence having at least 90%, preferably 95%, 96%, 97%, 98%, 99%, and 100% identity to the nucleotide sequence 5'-GAUACAA/X/UUCCAAAUACACAUA-3' (SEQ ID NO. 19); **X** is any of the modified nucleoside monomers; optionally, the sugar ring and phosphate ester bond of the double-stranded ribonucleic acid may be subjected to conventional chemical modifications to enhance stability.

In some embodiments, the double-stranded ribonucleic acid comprises: a sense strand consisting of a nucleotide sequence having at least 90%, preferably 95%, 96%, 97%, 98%, 99%, and 100% identity to the nucleotide sequence 5'-GUGAAGAAAUGUUGUUACGAU-3' (SEQ ID NO. 20); and an antisense strand consisting of a nucleotide sequence having at least 90%, preferably 95%, 96%, 97%, 98%, 99%, and 100% identity to the nucleotide sequence 5'-AUCGUAA/X/AACAUUUCUUCACUA-3' (SEQ ID NO. 21); **X** is any of the modified nucleoside monomers; optionally, the sugar ring and phosphate ester bond of the double-stranded ribonucleic acid may be subjected to conventional chemical modifications to enhance stability.

In some embodiments, **X** described above is selected from: M3C, 2M4C, M4C, and CP4C.

In some embodiments, the sugar ring chemical modification described above may be selected from a 2'-modification, and further may be selected from a 2'-F modification and a 2'-OMe modification.

In some embodiments, the dsRNA may be subjected to conventional chemical modifications (mainly conventional chemical modifications of the sugar ring and phosphate ester bond) to enhance stability. The chemical modification includes, but is not limited to, a sugar ring 2'-modification, and examples of the sugar ring 2'-modification include, but are not limited to: 2'-methoxyethyl, 2'-O-methyl (2'-OMe), 2'-O-allyl, 2'-C-allyl, 2'-deoxy, 2'-fluoro, 2'-O-N-methylacetamido (2'-O-NMA), 2'-O-dimethylaminoethoxyethyl (2'-O-DMAEOE), 2'-O-aminopropyl (2'-O-AP), 2'-fluoro, 2'-ara-F, and the like. Examples of conventional chemical modifications of a phosphate ester bond include, but are not limited to, the replacement of an internucleotide phosphate ester bond with other linking bonds such as a phosphorothioate bond. More than one such modification may be used.

In some embodiments, the double-stranded ribonucleic acid comprises:
Sense strand
   5'-AfsAmsUfGmAfCmUfUmUfUfAfUmUfGmAfGmCfUmCfUmUf-3' (SEQ ID NO.22)
Antisense strand:
   5'-AmsAfsGmAfGm/**X**/UmCfAmAfUmAmAmAfAmGfUmCfAmUfUmsCmsUm-3' (SEQ ID NO.23)
**X** is any of the modified nucleoside monomers.

In some embodiments, the double-stranded ribonucleic acid comprises:
Sense strand:
   5'-AfsAmsUfGmAfCmUfUmUfUfAfUmUfGmAfGmCfUmCfUmUf-3'(SEQ ID NO.22)
Antisense strand:
   5'-AmsAfsGmAfGmCfUm/**X**/AmAfUmAmAmAfAmGfUmCfAmUfUmsCmsUm-3'(SEQ ID NO.24)
**X** is any of the modified nucleoside monomers.

In some embodiments, the double-stranded ribonucleic acid comprises:
Sense strand:
   5'-CmsCmsAmCmAmAmCfGmCfUfUfUmUmGmGmGmGmGmUmGmAm-3'(SEQ ID NO.25)
Antisense strand:
   5'-UmsCfsAmCmCm/**X**/CmCmAmAmAmAmGmCfGmUfUmGmUmGmGmsGmsCm-3'(SEQ ID NO.26)
**X** is any of the modified nucleoside monomers.

In some embodiments, the double-stranded ribonucleic acid comprises:
Sense strand:
   5'-CmsCmsAmCmAmAmCfGmCfUfUfUmUmGmGmGmGmGmUmGmAm-3'(SEQ ID NO.25)
Antisense strand:
   5'-UmsCfsAmCmCmCf/**X**/CmAmAmAmAmGmCfGmUfUmGmUmGmGmsGmsCm-3'(SEQ ID NO.27)
**X** is any of the modified nucleoside monomers.

In some embodiments, the double-stranded ribonucleic acid comprises:
Sense strand:
   5'-CmsCmsAmCmAmAmCfGmCfUfUfUmUmGmGmGmGmGmUmGmAm-3'(SEQ ID NO.25)
Antisense strand:
   5'-UmsCfsAmCmCmCfCm/**X**/AmAmAmAmGmCfGmUfUmGmUmGmGmsGmsCm -3'(SEQ ID NO.28)
**X** is any of the modified nucleoside monomers.

In some embodiments, the double-stranded ribonucleic acid comprises:
Sense strand:
   5'- GmsAmsGmAmAmUmUfUmUfGfGfUmUmGmGmGmCmCmUmAmGm-3' (SEQ ID NO.29)
Antisense strand:
   5'-CmsUfsAmGmGm/**X**/CmCmAmAmCmCmAmAfAmAfUmUmCmUmCmsCmsAm-3' (SEQ ID NO.30)
**X** is any of the modified nucleoside monomers.

In some embodiments, the double-stranded ribonucleic acid comprises:
Sense strand:
   5'- GmsAmsGmAmAmUmUfUmUfGfGfUmUmGmGmGmCmCmUmAmGm-3' (SEQ ID NO.29)
Antisense strand:
   5'-CmsUfsAmGmGmCf/**X**/CmAmAmCmCmAmAfAmAfUmUmCmUmCmsCmsAm-3' (SEQ ID NO.31)
**X** is any of the modified nucleoside monomers.

In some embodiments, the double-stranded ribonucleic acid comprises:
Sense strand:
   5'- GmsAmsGmAmAmUmUfUmUfGfGfUmUmGmGmGmCmCmUmAmGm-3' (SEQ ID NO.29)
Antisense strand:
   5'-CmsUfsAmGmGmCfCm/**X**/AmAmCmCmAmAfAmAfUmUmCmUmCmsCmsAm-3' (SEQ ID NO.32)
**X** is any of the modified nucleoside monomers.

In some embodiments, the double-stranded ribonucleic acid comprises:
Sense strand:
   5'-CmsCmsCmAmGmUmGfUmCfAfGfGmUmGmGmGmAmGmUmAmCm-3' (SEQ ID NO.33)
Antisense strand:
   5'-GmsUfsAmCmUm/**X**/CmCmAmCmCmUmGmAfCmAfCmUmGmGmGmsAmsUm-3' (SEQ ID NO.34)
**X** is any of the modified nucleoside monomers.

In some embodiments, the double-stranded ribonucleic acid comprises:
Sense strand:
   5'-CmsCmsCmAmGmUmGfUmCfAfGfGmUmGmGmGmAmGmUmAmCm-3' (SEQ ID NO.33)
Antisense strand:
   5'-GmsUfsAmCmUmCf/**X**/CmAmCmCmUmGmAfCmAfCmUmGmGmGmsAmsUm-3' (SEQ ID NO.35)
**X** is any of the modified nucleoside monomers.

In some embodiments, the double-stranded ribonucleic acid comprises:
Sense strand:
   5'-CmsCmsCmAmGmUmGfUmCfAfGfGmUmGmGmGmAmGmUmAmCm-3' (SEQ ID NO.33)
Antisense strand:
   5'-GmsUfsAmCmUmCfCm/**X**/AmCmCmUmGmAfCmAfCmUmGmGmGmsAmsUm-3' (SEQ ID NO.36)
**X** is any of the modified nucleoside monomers.

In some embodiments, the double-stranded ribonucleic acid comprises:
Sense strand:
   5'-GmsUmsGmAmUmUmCfAmAfGfUfUmUmAmUmGmGmAmUmAmCm-3' (SEQ ID NO.37)
Antisense strand:
   5'-GmsUfsAmUmCm/**X**/AmUmAmAmAmCmUmUfGmAfAmUmCmAmCmsAmsAm-3' (SEQ ID NO.38)
**X** is any of the modified nucleoside monomers.

In some embodiments, the double-stranded ribonucleic acid comprises:
Sense strand:
   5'-UmsGmsUmGmUmAmUfUmUfGfGfAmAmGmUmUmGmUmAmUmCm -3' (SEQ ID NO.39)
Antisense strand:
   5'-GmsAfsUmAmCmAmAm/**X**/UmUmCmCmAmAfAmUfAmCmAmCmAmsUmsAm -3' (SEQ ID NO.40)
**X** is any of the modified nucleoside monomers.

In some embodiments, the double-stranded ribonucleic acid comprises:
Sense strand:
   5'- GmsUmsGmAmAmGmAfAmAfUfGfUmUmGmUmUmAmCmGmAmUm -3' (SEQ ID NO.41)
Antisense strand:
   5'-AmsUfsCmGmUmAmAm/**X**/AmAmCmAmUmUfUmCfUmUmCmAmCmsUmsAm -3' (SEQ ID NO.42)
**X** is any of the modified nucleoside monomers.

In some embodiments, **X** is selected from: M3C, 2M4C, M4C, and CP4C.

In some embodiments, the sugar ring of **X** described above may be further modified, including: a 2'-F modification and a 2'-Ome modification.

The dsRNA of the present disclosure can also be expressed from a recombinant virus vector in a cell *in vivo.* The recombinant virus vectors of the present disclosure comprise a sequence encoding the dsRNA of the present disclosure and any promoter suitable for expressing the dsRNA sequence. Suitable promoters include, for example, U6 or H1 RNA pol III promoter sequence and the cytomegalovirus promoter. The selection of other suitable promoters is within the skill in the art. The recombinant virus vectors of the present disclosure can also contain an inducible or regulatable promoter to express the dsRNA in specific tissues or in specific intracellular environments.

The dsRNA of the present disclosure can be expressed from a recombinant virus vector as two separate complementary RNA molecules or as a single RNA molecule with two complementary regions. Any virus vector capable of accepting the encoding sequence for expressing a dsRNA molecule can be used, for example vectors derived from adenovirus (AV), adeno-associated virus (AAV), retrovirus (e.g., lentivirus (LV), rhabdovirus, and murine leukemia virus), herpes virus, and the like. The tropism of the virus vector can be altered by pseudotyping the virus vector using envelope proteins or other surface antigens from other viruses or by substituting different viral capsid proteins, as appropriate.

### Definitions:

Unless otherwise specified, the term "alkyl" refers to a monovalent saturated aliphatic hydrocarbon group, i.e., a linear or branched group containing 1 to 20 carbon atoms, preferably containing 1 to 10 carbon atoms (i.e., C1-10 alkyl), further preferably containing 1 to 8 carbon atoms (C1-8 alkyl), and more preferably containing 1 to 6 carbon atoms (i.e., C1-6 alkyl), for example, "C1-6 alkyl" means that the group is alkyl and the number of carbon atoms on the carbon chain is between 1 and 6 (specifically 1, 2, 3, 4, 5, or 6). Examples of the alkyl include, but are not limited to, methyl, ethyl, *n*-propyl, isopropyl, *n*-butyl, isobutyl, *tert-*butyl, *sec-*butyl, *n*-pentyl, neopentyl, 1,1-dimethylpropyl, 1,2-dimethylpropyl, 2,2-dimethylpropyl, 1-ethylpropyl, 2-methylbutyl, 3-methylbutyl, *n*-hexyl, *n-*heptyl, *n*-octyl, and the like. The term "alkyl chain" refers to a structure in which both ends of a linear or branched chain of carbon atoms of a saturated aliphatic hydrocarbon are connected to other atoms. Unless otherwise specified, the term "cycloalkyl" refers to a monocyclic saturated aliphatic hydrocarbon group containing a specific number of carbon atoms, preferably containing 3 to 12 carbon atoms (i.e., C3-12 cycloalkyl), more preferably containing 3 to 10 carbon atoms (C3-10 cycloalkyl), and further preferably containing 3 to 6 carbon atoms (C3-6 cycloalkyl), 4 to 6 carbon atoms (C4-6 cycloalkyl), or 5 to 6 carbon atoms (C5-6 cycloalkyl). Examples of the cycloalky include, but are not limited to, cyclopropyl, cyclobutyl, cyclopentyl, cyclohexyl, methylcyclopropyl, 2-ethylcyclopentyl, dimethylcyclobutyl, and the like.

Unless otherwise specified, the term "alkoxy" refers to -O-alkyl, wherein the alkyl is defined as above, i.e., the alkyl contains 1 to 20 carbon atoms, preferably 1 to 10 carbon atoms, more preferably 1 to 8 carbon atoms, and further more preferably 1 to 6 carbon atoms (specifically 1, 2, 3, 4, 5, or 6). Representative examples of the alkoxy include, but are not limited to, methoxy, ethoxy, propoxy, isopropoxy, butoxy, 1-methylpropoxy, 2-methylpropoxy, *tert*-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, and the like.

Unless otherwise specified, the term "halogen" or "halo" refers to F, Cl, Br, or I. The term "haloalkyl" means that one, two, or more hydrogen atoms or all hydrogen atoms in the alkyl defined as above are substituted with halogen. Representative examples of the haloalkyl include CCl₃, CF₃, CHCl₂, CH₂Cl, CH₂Br, CH₂I, CH₂CF₃, CF₂CF₃, and the like.

Unless otherwise specified, the term "heterocyclyl" refers to a saturated or partially unsaturated monocyclic, bicyclic, or polycyclic hydrocarbon substituent, and has a non-aromatic structure containing 3 to 20 ring atoms, wherein 1, 2, 3, or more ring atoms are selected from N, O, and S, and the remaining ring atoms are C. Preferably, 3-12 ring atoms are contained; further preferably 3-10 ring atoms, or 3-8 ring atoms, or 3-6 ring atoms, or 4-6 ring atoms, or 5-6 ring atoms are contained. The number of the heteroatoms is preferably 1-4, more preferably 1-3 (i.e., 1, 2, or 3). Examples of the monocyclic heterocyclyl include pyrrolidinyl, imidazolidinyl, tetrahydrofuranyl, dihydropyrrolyl, piperidyl, piperazinyl, pyranyl, and the like. Polycyclic heterocyclyl includes spiroheterocyclyl, fused heterocyclyl, and bridged heterocyclyl. "Heterocycloalkyl" refers to a saturated "heterocyclyl" described above.

Unless otherwise specified, the term "aryl" refers to a monocyclic, bicyclic and tricyclic aromatic carbocyclic system containing 6 to 16 carbon atoms, or 6 to 14 carbon atoms, or 6 to 12 carbon atoms, or 6 to 10 carbon atoms, preferably 6 to 10 carbon atoms, and the term "aryl" is used interchangeably with the term "aromatic cyclyl". Examples of the aryl group may include, but are not limited to, phenyl, naphthyl, anthryl, phenanthryl, pyrenyl, or the like.

Unless otherwise specified, the term "heteroaryl", "heteroaromatic ring group", and "aromatic heterocyclic ring group" refer to an aromatic monocyclic or polycyclic ring system containing a 5- to 12-membered structure, or preferably a 5- to 10-membered structure, or a 5- to 8-membered structure, and more preferably a 5- to 6-membered structure, wherein 1, 2, 3, or more ring atoms are heteroatoms and the remaining atoms are carbon, the heteroatoms are independently selected from O, N, and S, and the number of the heteroatoms is preferably 1, 2, or 3. Examples of the heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolyl, isoquinolyl, pteridinyl, purinyl, indolyl, isoindolyl, indazolyl, benzofuranyl, benzothienyl, benzopyridyl, benzopyrimidinyl, benpyrazinyl, benzimidazolyl, benzophthalizinyl, pyrrolo[2,3-b]pyridyl, imidazo[1,2-a]pyridyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, and the like.

Unless otherwise specified, the term "amino protective group" refers to a moiety that temporarily blocks the amine-reactive site in the compound. Preferably, the amino protective group is selectively removable by a chemical reaction, if desired, using methods well known in the art. Common amino protective groups include, but are not limited to: benzoyl (Bz), acetyl (Ac), benzyloxycarbonyl (CBz), *tert*-butoxycarbonyl (Boc), 9-fluorenylmethoxycarbonyl (Fmoc), *p*-methoxybenzyl (PMB), benzyl (Bn), trityl (Tr), *p*-toluenesulfonyl (Tos), phthaloyl (Pht), allyloxycarbonyl (Alloc), and trifluoroacetyl (Tfa). The amino protective agent is further preferably benzoyl (Bz), acetyl (Ac), benzyloxycarbonyl (CBz), *tert*-butoxycarbonyl (Boc), and 9-fluorenylmethoxycarbonyl (Fmoc).

Unless otherwise specified, the term "hydroxyl protecting group" refers to a moiety that temporarily blocks the hydroxy-reactive site in the compound. Preferably, the hydroxyl protecting group is selectively removable by a chemical reaction, if desired, using methods well known in the art. Common hydroxyl protecting groups for use include, but are not limited to, methoxymethyl ether (MOM), 2-tetrahydropyran (THP), *tert*-butyldimethylsilyl chloride (TBS), trimethylsilyl (TMS), triethylsilyl (TES), trityl (Tr), 4,4'-dimethoxytrityl (DMTr), 4,4',4'-trimethoxytrityl (TMTr), benzyl (Bn), benzoyl (Bz), acetyl (Ac), and *p*-methoxybenzyl (PMB). Further preferably, the hydroxyl protecting group is trityl (Tr), 4,4'-dimethoxytrityl (DMTr), 4,4',4'-trimethoxytrityl (TMTr), benzyl (Bn), benzoyl (Bz), or acetyl (Ac).

Generally, "G", "C", "A", and "U" represent nucleotides comprising guanine, cytosine, adenine, and uracil as bases, respectively. However, it is to be understood that the term "ribonucleotide" or "nucleotide" may also refer to a modified nucleotide explicitly described hereinafter, or a surrogate replacement moiety. It is well known to those skilled that guanine, cytosine, adenine, and uracil can be replaced by other moieties without substantially altering the base pairing properties of an oligonucleotide comprising nucleotides bearing such replacement moieties. Sequences comprising such replacement moieties are embodiments of the present disclosure.

"PCSK9" as used herein refers to the gene or protein of proprotein convertase subtilisin/kexin type 9 (also known as FH3, HCHOLA3, NARC-1, or NARC1). Provided mRNA sequences of PCSK9 include human: NM_174936; mouse: NM_153565; and rat: NM_199253.

"Target gene" as used herein is not limited to PCSK9, and all existing genes that can be regulated by siRNA fall within the scope of the target gene defined by the present disclosure. For example, the target gene may be PCSK9, ANGPTL3, FXII, LPA, and APOB genes, preferably ANGPTL3 and PCSK9 genes, and most preferably PCSK9 gene.

"LPA" as used herein refers to LP(A), also known as lipoprotein a, which is synthesized primarily in the liver. The protein encoded by the gene is a serine protease that is capable of inhibiting the activity of tissue-type plasminogen activator I.

"FXII" as used herein refers to coagulation factor 12, one of the coagulation factors, and its deficiency results in prolonged time of venous blood coagulation.

"APOB" as used herein refers to apolipoprotein B, a protein in plasma lipoproteins, and its basic function is carrying lipids. It may be divided into the following subclasses: apoB48 and apoB100. ApoB48 is one of the apolipoproteins for chylomicrons (CM); apoB100 is one of the apolipoproteins for very low-density lipoprotein (VLDL) and low-density lipoprotein (LDL).

Unless otherwise stated, when used to describe the relationship between a first nucleotide sequence and a second nucleotide sequence, the term "complementary" as used herein refers to the ability of an oligonucleotide or polynucleotide comprising the first nucleotide sequence to hybridize with an oligonucleotide or polynucleotide comprising the second nucleotide sequence under certain conditions and form a double-helix structure, which can be understood by those skilled in the art. For example, such conditions may be strict conditions, including 400 mM NaCl, 40 mM PIPES pH 6.4, 1 mM EDTA, sustaining a temperature of 50 °C or 70 °C for 12-16 hours, and then performing subsequent washing. Other conditions, such as physiologically relevant conditions that may be encountered in an organism, may also be applicable. Those skilled in the art are able to determine a series of conditions most suitable for the complementation assay of two sequences on the basis of the ultimate application of the hybridized nucleotides.

The conditions include base pairing of an oligonucleotide or polynucleotide comprising the first nucleotide sequence with an oligonucleotide or polynucleotide comprising the second nucleotide sequence over the full length of the first and second nucleotide sequences. Herein, these sequences are referred to as "fully complementary" to each other. However, as mentioned herein, when the first sequence is "substantially complementary" to the second sequence, the two sequences may be fully complementary or form one or more, but generally, not more than 4, 3, or 2 mismatched base pairs upon hybridization, while retaining the ability to hybridize under the conditions most relevant to their ultimate application. However, when two oligonucleotides are designed to form one or more single-stranded overhangs upon hybridization, such overhangs should not be considered a mismatch given the definition of complementarity. For example, in a dsRNA comprising one oligonucleotide of 21 nucleotides in length and another oligonucleotide of 23 nucleotides in length, the longer oligonucleotide comprises a sequence of 21 nucleotides in length fully complementary to the shorter oligonucleotide, which, in this case, also belongs to "fully complementary" for the purposes of the present disclosure.

The term "double-stranded ribonucleic acid", "double-stranded RNA", or "dsRNA" as used herein refers to a complex of ribonucleic acid molecules; the complex has a double-stranded structure and comprises two anti-parallel and substantially complementary nucleic acid strands as described above. The two strands forming the double-stranded structure may be different portions of the same relatively large RNA molecule, or may be separate RNA molecules. If the two strands are separate RNA molecules, such dsRNAs are commonly referred to as siRNAs ("small interfering RNAs") in literature. If the two strands are portions of a relatively large molecule and are connected by a non-interrupted nucleotide strand forming a double-stranded structure between the 3'-end of one strand and the 5'-end of the other strand, the connected RNA strands are referred to as a "hairpin loop", "short hairpin RNA", or "shRNA". If the two strands are covalently connected by other means than a non-interrupted strand forming a double-stranded structure between the 3'-end of one strand and the 5'-end of the other strand, the connection structure is referred to as a "linker". The RNA strands may have identical or different numbers of nucleotides. The maximum number of base pairs is the number of nucleotides in the shortest strand in a dsRNA subtracted by any overhangs present in the double strand. In addition to a double-stranded structure, a dsRNA may include one or more nucleotide overhangs. In addition, "dsRNA" as used in this specification may include chemical modifications on ribonucleotides, including modifications on multiple nucleotides and all types of modifications disclosed herein or known in the art. For the purposes of this specification and claims, "dsRNA" encompasses all such modifications used for siRNA-type molecules.

The terms "dsRNA," "siRNA," and "iRNA reagent" may be used interchangeably for reagents that are able to mediate silencing of a target RNA (e.g., an mRNA, such as a transcript of a protein-encoding gene). For convenience, such mRNAs are also referred to as mRNAs to be silenced herein. Such a gene is also referred to as a target gene. Generally, the RNA to be silenced is an endogenous gene or a pathogen gene. In addition, RNAs other than mRNAs (e.g., tRNAs) and viral RNAs may also be targeted.

"Nucleotide overhang" as used herein refers to one or more unpaired nucleotides that protrude from the double-stranded structure of a dsRNA when the 3'-end of one strand exceeds the 5'-end of the other strand of the dsRNA, or vice versa. "Blunt end" or "blunt terminal" refers to no unpaired nucleotides at the ends of a dsRNA, i.e., no nucleotide overhangs. A "blunt-ended" dsRNA refers to a dsRNA that is double-stranded over its entire length, i.e., no nucleotide overhang at any end of the molecule. For clarity, while determining whether an siRNA has overhangs or blunt ends, a chemical cap or non-nucleotide chemical moiety bound to the 3'-end or 5'-end of the siRNA is not considered. The term "antisense strand" refers to one strand in a dsRNA, which comprises a region substantially complementary to a target sequence. The term "complementarity region" as used herein refers to a region on the antisense strand that is substantially complementary to a sequence defined herein (e.g., a target sequence). If the complementarity region is not fully complementary to the target sequence, the most tolerable mismatch occurs in the terminal regions, and if a mismatch exists, it is typically located in the terminal regions or, for example, within 6, 5, 4, 3, or 2 nucleotides of the 5' end and/or 3' end.

The term "sense strand" as used herein refers to one strand in a dsRNA, which comprises a region substantially complementary to the antisense strand region.

The "conventional chemical modification" described in the present disclosure on a dsRNA mainly includes conventional chemical modifications on sugar rings and phosphate ester bonds for enhancing stability. The chemical modification includes, but is not limited to, a sugar ring 2'-modification, and examples of the sugar ring 2'-modification include, but are not limited to: 2'-methoxyethyl, 2'-O-methyl (2'-OMe), 2'-O-allyl, 2'-C-allyl, 2'-deoxy, 2'-fluoro, 2'-O-N-methylacetamido (2'-O-NMA), 2'-O-dimethylaminoethoxyethyl (2'-O-DMAEOE), 2'-O-aminopropyl (2'-O-AP), 2'-fluoro, 2'-ara-F, and the like. Examples of conventional chemical modifications of a phosphate ester bond include, but are not limited to, the replacement of an internucleotide phosphate ester bond with other linking bonds such as a phosphorothioate bond. More than one such modification may be used.

As understood by those skilled in the art, when a dsRNA is "introduced into a cell (or introduced to a cell)", the case refers to facilitating the uptake or absorption into the cell. The absorption or uptake of a dsRNA may occur by means of non-facilitated diffusion or active cellular processes, or by means of assisting reagents or devices. The connotation of this term is not limited to cells *in vitro*; a dsRNA may also be "introduced into a cell (or introduced to a cell)", wherein the cell is part of a living organism. In this case, introduction into the cell will include delivery to the organism. For example, in *in vivo* delivery, the dsRNA may be injected into a tissue site or administered systemically. Introduction to cells *in vitro* includes methods known in the art, such as electroporation and lipofection.

"Pharmaceutical composition" as used herein comprises a pharmacologically effective amount of a dsRNA and a pharmaceutically acceptable carrier. "Pharmacologically effective amount", "therapeutically effective amount", or "effective amount" as used herein refers to an RNA amount effective to produce a desired pharmacological, therapeutic, or prophylactic result. For example, if a given clinical treatment that reduces a measurable parameter associated with a disease or disorder by at least 25% is considered as an effective treatment, a therapeutically effective amount of a drug used for treating the disease or disorder is the amount necessary to reduce the parameter by at least 25%. The term "pharmaceutically acceptable carrier" refers to a carrier used for administering a therapeutic agent. Such carriers include, but are not limited to, salt solutions, buffering salt solutions, glucose, water, glycerol, ethanol, and combinations thereof.

The term "silencing" refers to at least partial suppression of a target gene expression.

### BRIEF DESCRIPTION OF THE DRAWINGS

FIG. 1 is the plot of change in gene numbers down-regulated and up-regulated by the modified dsRNAs and controls.
FIG. 2 is the retention effect (%) of the modified dsRNAs on the target mRNA (PCSK9).
FIG. 3 is the retention effect (%) of the modified dsRNAs on the target mRNA (ANGPTL3).
FIG. 4 is the retention effect (%) of the modified dsRNAs on the target mRNA (LPA).
FIG. 5 is the plot of change in gene numbers down-regulated and up-regulated by the modified dsRNAs (targeting the C5 gene) and controls.
FIG. 6 is the retention effect (%) of the modified dsRNAs on the target mRNA (C5).

### Examples:

| Abbreviation | **Corresponding meaning** |
|---|---|
| f | 2'-Fluoro |
| m | 2'-O-methyl |
| s | Replacement of phosphate ester of the linking moiety by thiophosphate ester |
| Afs | 2'-Fluoroadenosine (with the labeled s, the abbreviation indicates that the 3' position in the sequence is linked by thiophosphate ester; the same applies hereinafter) |
| Af | 2'-Fluoroadenosine (without the labeled s, the abbreviation indicates that the 3' position in the sequence is linked by phosphate ester; the same applies hereinafter) |
| Am | 2'-O-methyladenosine |
| Ams | 2'-O-methyladenosine (the 3' position in the sequence is linked by thiophosphate ester) |
| Uf | 2'-Fluorouridine |
| Um | 2'-O-methyluridine |
| Ums | 2'-O-methyluridine (the 3' position in the sequence is linked by thiophosphate ester) |
| Gm | 2'-O-methylguanosine |
| Gf | 2'-Fluoroguanosine |
| Cm | 2'-O-methylcytidine |
| Cms | 2'-O-methylcytidine (the 3' position in the sequence is linked by thiophosphate ester) |
| Cf | 2'-Fluorocytidine-3'-phosphate |
| 2'-O-Methyl | 2'-O-methyl |
| Thiophosphate | Thiophosphate |
| 2'-fluoro | 2'-Fluoro |
| Blank | Blank control |
| NC | Negative control (the sequence does not cause changes in gene expression) |
| PC | Positive control or modification control |

The present disclosure will be further illustrated with reference to the following specific examples. It should be understood that these examples are merely intended to illustrate the present disclosure rather than limit the scope of the present disclosure. Experimental procedures without specified conditions in the following examples, are generally conducted according to conventional conditions or according to conditions recommended by the manufacturer. Unless otherwise defined, all technical and scientific terms used herein have the same meaning as commonly understood by those of ordinary skill in the art. In addition, any methods and materials similar or equivalent to those described herein can be used in the methods of the present disclosure. The preferred embodiments and materials described herein are for illustrative purposes only.

### Example 1: Synthesis of Modified Nucleoside Monomer Compounds

### 1.1 Synthesis of 3-N-methyl-4-N-benzoyl-5'-(O-4,4'-dimethoxytrityl)-2'-fluoro-2'-deoxycytidine, 3'-[(2-cyanoethyl)-(N,N-diisooropyl)1-phosphoramidite (5):

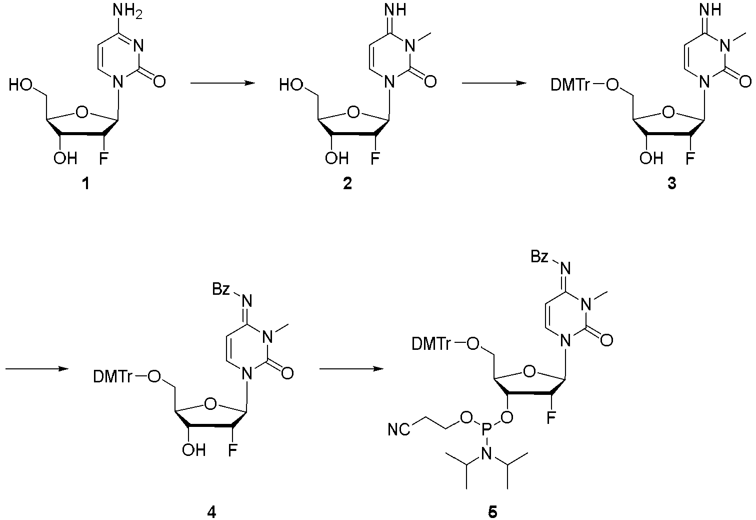

### Synthesis of 3-N-methyl-2'-fluoro-2'-deoxycytidine (2)

After 2'-fluoro-2'-deoxyuridine (**1**) (3.68 g, 15 mmol) was dissolved in dry DMF (40 mL), iodomethane (1.9 mL, 30.3 mmol) was added. The reaction solution was stirred at room temperature for 24 h before DMF was removed. The remaining crude product formed an azeotrope with toluene (2 × 80 mL) to remove the residual DMF and then reconstituted in 15 mL of acetone. *n*-Hexane (40 mL) was added, and the mixture was left to stand at -20 °C for 1 h. The resulting precipitate was filtered, and the filter cake was washed with cold acetone/n-hexane and dried under vacuum to give compound **2** (2.57 g, 9.9 mmol; yield: 66%) as a light yellow solid. HRMS (ESI-TOF) [M+H]⁺ = 260.0970.

### 3-N-Methyl-5'-(O-4,4'-dimethoxytrityl)-2'-fluoro-2'-deoxycytidine (3)

After compound **2** (2.4 g, 9.3 mmol) was dissolved in 30 mL of dry pyridine, 4,4'-dimethoxytrityl chloride (4.7 g, 14.0 mmol) was added. The reaction solution was stirred at room temperature overnight before 200 mL of dichloromethane was added. The organic phase was serially washed with 5% aqueous Na₂S₂O₃ solution (2 × 80 mL), saturated sodium carbonate (80 mL), and saturated brine (80 mL), then dried over anhydrous sodium sulfate, filtered, and concentrated under reduced pressure to remove the solvent. The remaining crude product was separated by column chromatography to give compound 3 (2.4 g, 4.3 mmol; yield: 46%) as a white solid. HRMS (ESI-TOF) [M+H]⁺ = 562.2281.

### 3-N-Methyl-4-N-benzoyl-5'-(O-4,4'-dimethoxytrityl)-2'-fluoro-2'-deoxycytidine (4)

Compound **3** (2.3 g, 4.1 mmol) was distilled under reduced pressure with pyridine (2 × 50 mL) to remove excessive water, and then reconstituted in 50 mL of dry pyridine. After trimethylsilyl chloride (TMSCl) (2.1 mL, 16.4 mmol) was added, the mixture was stirred at room temperature for 1.5 hours, and benzoyl chloride

(BzCl; 0.69 g, 4.92 mmol) was added. The mixture was successively stirred at room temperature for 4 hours. After the reaction was complete, 10 mL of water was added, and the mixture was stirred at room temperature for 5 min, followed by the addition of aqueous ammonia (15 mL, 25-28% w/w). The reaction mixture was successively stirred at room temperature for 15 min followed by distillation under reduced pressure to remove the solvent. The remaining crude product was separated by column chromatography to give compound **4** (2.1 g, 3.20 mmol; yield: 78%) as a white solid. HRMS (ESI-TOF) [M+H]⁺ = 666.2546.

### 3-N-Methyl-4-N-benzoyl-5'-(O-4,4'-dimethoxytrityl)-2'-fluoro-2'-deoxycytidine, 3'-[(2-cyanoethyl)-(N,N-diisopropyl)]-phosphoramidite (5)

After compound **4** (533 mg, 0.8 mmol) was dissolved in 15 mL of dry dichloromethane, *N,N-*diisopropylethylamine (0.40 mL, 3.2 mmol) and 2-cyanoethyl-*N,N*-diisopropylphosphoramidite chloride (0.24 mL, 1.60 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **5** (408 mg, 0.48 mmol; yield: 60%) as a white solid. HRMS (ESI-TOF) [M+H]⁺ = 866.3611.

### 1.2 Synthesis of 4-N-methyl-5'-(O-4,4'-dimethoxytrityl)-2'-fluoro-2'-deoxycytidine, 3'-[(2-cyanoethyl)-(N,N-diisopropyl)]-phosphoramidite (10)

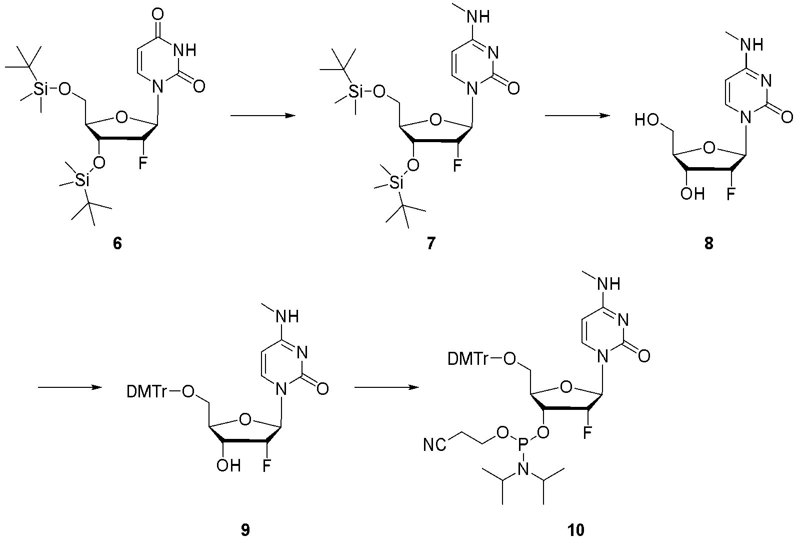

### 4-N-methyl-3',5'-di-tert-butyldimethylsilyl-2'-fluoro-2'-deoxycytidine (7)

Compound **6** (3.80 g, 8.0 mmol) was added to a sealed tube and dissolved in 100 mL of dry acetonitrile. After the reaction mixture was cooled to 0 °C, triethylamine (3.24 g, 32.0 mmol), p-toluenesulfonyl chloride (3.05 g, 16.0 mmol), and 4-dimethylaminopyridine (1.95 g, 16.0 mmol) were separately added. The reaction mixture was stirred at 0 °C for 2-3 hours followed by the addition of an aqueous methylamine solution (320 mL, 25%-30% in water). After the sealed tube was sealed, the mixture was heated to 50 °C, stirred for 48 h, cooled to room temperature, and concentrated under reduced pressure. The resulting crude product was separated by column chromatography to give compound **7** (1.80 g, 3.68 mmol; yield: 46%) as a white solid. HRMS (ESI-TOF) [M+H]⁺ = 487.2704.

### 4-N-Methyl-2'-fluoro-2'-deoxycytidine (8)

After compound **7** (1.6 g, 3.28 mmol) was dissolved in 17 mL of tetrahydrofuran, tetrabutylammonium fluoride (TBAF) (7.2 mL, 1 M in THF) was added. The reaction mixture was stirred at room temperature for 16 hours and then concentrated to give a crude product, which was separated by column chromatography to give compound **8** (0.55 g, 2.13 mmol; yield: 65%) as a white solid. HRMS (ESI-TOF) [M+H]⁺ = 260.0972.

### 4-N-Methyl-5'-(O-4,4'-dimethoxytrityl)-2'-fluoro-2'-deoxycytidine (9)

After compound **8** (500 mg, 1.93 mmol) was dissolved in 6.5 mL of dry pyridine, 4,4'-dimethoxytrityl chloride (0.97 g, 2.90 mmol) was added. The reaction mixture was stirred at room temperature overnight followed by dilution with 40 mL of dichloromethane. The organic phase was serially washed with 5% aqueous Na₂S₂O₃ solution (2 × 20 mL), saturated aqueous sodium carbonate solution (20 mL), and saturated brine (20 mL). The organic phase was collected, dried over anhydrous sodium sulfate, and concentrated to give a crude product, which was separated by column chromatography to give compound **9** (552.8 mg, 0.98 mmol; yield: 51%) as a white solid. HRMS (ESI-TOF) [M+H]⁺ = 562.2281.

### 4-N-Methyl-5'-(O-4,4'-dimethoxytrityl)-2'-fluoro-2'-deoxycytidine, 3'-[(2-cyanoethyl)-(N,N-diisopropyl)]-phosphoramidite (10)

After compound **9** (449.3 mg, 0.8 mmol) was dissolved in 15 mL of dry dichloromethane, *N,N-*diisopropylethylamine (0.40 mL, 3.2 mmol) and 2-cyanoethyl-*N,N*-diisopropylphosphoramidite chloride (0.24 mL, 1.60 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **10** (370.0 mg, 0.50 mmol; yield: 62%) as a white solid. HRMS (ESI-TOF) [M+H]⁺ = 762.3351.

### 1.3 Synthesis of 4-N,N-dimethyl-5'-(O-4,4'-dimethoxytrityl)-2'-fluoro-2'-deoxycytidine, 3'-[(2-cyanoethyl)-(N,N-diisopropyl)]-phosphoramidite (14)

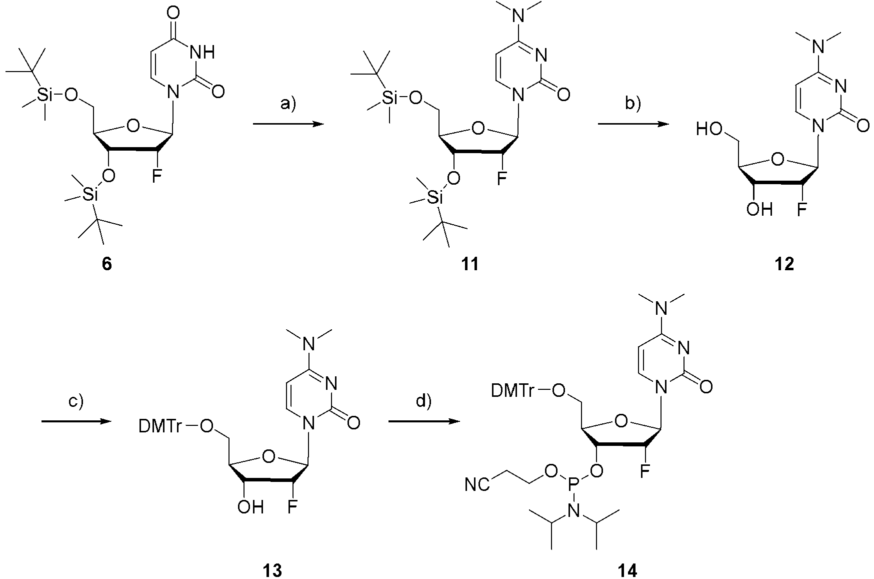

### 4-N,N-Dimethyl-3',5'-di-tert-butyldimethylsilyl-2'-fluoro-2'-deoxycytidine (11)

In a sealed tube, compound **6** (3.80 g, 8.0 mmol) was dissolved in 100 mL of dry acetonitrile. After the mixture was cooled to 0 °C, triethylamine (3.24 g, 32.0 mmol), p-toluenesulfonyl chloride (3.05 g, 16.0 mmol), and 4-dimethylaminopyridine (1.95 g, 16.0 mmol) were separately added. The reaction mixture was stirred at 0 °C for 2-3 hours followed by the addition of 200 mL of aqueous dimethylamine solution (40%). After the sealed tube was sealed, the mixture was heated to 50 °C and stirred for 48 hours. The mixture was cooled to room temperature and concentrated under reduced pressure. The resulting crude product was separated by column chromatography to give compound **11** (2.33 g, 4.64 mmol; yield: 58%) as a white solid. HRMS (ESI-TOF) [M+H]⁺ = 502.2863.

### 4-N,N-Dimethyl-2'-fluoro-2'-deoxycytidine (12)

After compound **11** (2.0 g, 4.0 mmol) was dissolved in 20 mL of tetrahydrofuran, THF (20 mL) was added to tetrabutylammonium fluoride (TBAF) (8.8 mL, 1 M in THF). The reaction mixture was stirred at room temperature for 16 hours and then concentrated to give a crude product, which was separated by column chromatography to give compound **12** (961.9 mg, 3.5 mmol; yield: 88%) as a white solid. HRMS (ESI-TOF) [M+H]⁺ = 273.1117.

### 4-N,N-Dimethyl-5'-(O-4,4'-dimethoxytrityl)-2'-fluoro-2'-deoxycytidine (13)

After compound **12** (527.4 mg, 1.93 mmol) was dissolved in 6.5 mL of dry pyridine, 4,4'-dimethoxytrityl chloride (0.97 g, 2.90 mmol) was added. The reaction mixture was stirred at room temperature overnight followed by dilution with 40 mL of dichloromethane. The organic phase was serially washed with 5% aqueous Na₂S₂O₃ solution (2 × 20 mL), saturated aqueous sodium carbonate solution (20 mL), and saturated brine (20 mL). The organic phase was collected, dried over anhydrous sodium sulfate, and concentrated to give a crude product, which was separated by column chromatography to give compound **13** (722.1 mg, 1.25 mmol; yield: 65%) as a white solid. Molecular formula: C₃₂H₃₄FN₃O₆; HRMS (ESI-TOF) [M+H]⁺ = 576.2439.

### 4-N,N-dimethyl-5'-(O-4,4'-dimethoxytrityl)-2'-fluoro-2'-deoxycytidine, 3'-[(2-cyanoethyl)-(N,N-diisopropyl)]-phosphoramidite (14)

After compound **13** (460.5 mg, 0.8 mmol) was dissolved in 15 mL of dry dichloromethane, *N,N-*diisopropylethylamine (0.40 mL, 3.2 mmol) and 2-cyanoethyl-*N,N*-diisopropylphosphoramidite chloride (0.24 mL, 1.60 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **14** (413.3 mg, 0.54 mmol; yield: 68%) as a white solid. HRMS (ESI-TOF) [M+H]⁺ = 776.3521.

### 1.4 Synthesis of 4-N,N-dimethyl-5'-(O-4,4'-dimethoxytrityl)-2'-methoxycytidine, 3'-[(2-cyanoethyl)-(N,N-diisopropyl)]-chosphoramidite (19)

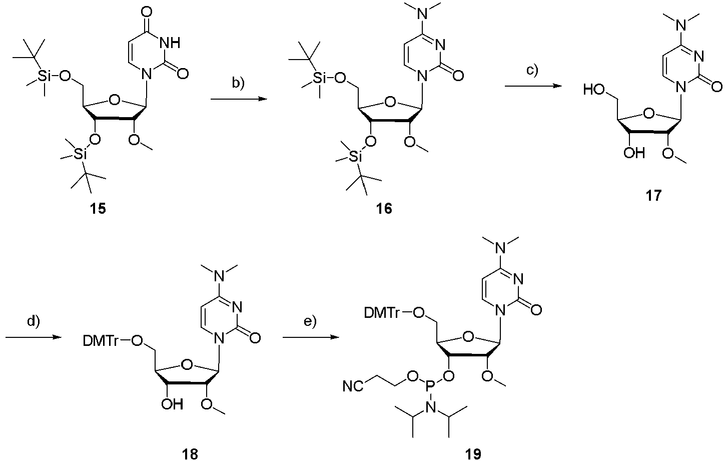

### 4-N,N-Dimethyl-3',5'-di-tert-butyldimethylsilyl-2'-methoxycytidine (16)

In a sealed tube, compound **15** (3.89 g, 8.0 mmol) was dissolved in 100 mL of dry acetonitrile. After the mixture was cooled to 0 °C, triethylamine (3.24 g, 32.0 mmol), *p*-toluenesulfonyl chloride (3.05 g, 16.0 mmol), and 4-dimethylaminopyridine (1.95 g, 16.0 mmol) were separately added. The reaction mixture was stirred at 0 °C for 2-3 hours followed by the addition of 200 mL of aqueous dimethylamine solution (40% in water). After the sealed tube was sealed, the mixture was heated to 50 °C and stirred for 48 hours. The mixture was cooled to room temperature and concentrated under reduced pressure. The resulting crude product was separated by column chromatography to give compound **16** (2.50 g, 4.88 mmol; yield: 61%) as a white solid. HRMS (ESI-TOF) [M+H]⁺ = 514.3081.

### 4-N,N-Dimethyl-2'-methoxycytidine (17)

After compound **16** (2.05 g, 4.0 mmol) was dissolved in 20 mL of tetrahydrofuran, THF (20 mL) was added to tetrabutylammonium fluoride (TBAF) (8.8 mL, 1 M in THF). The reaction mixture was stirred at room temperature for 16 hours and then concentrated to give a crude product, which was separated by column chromatography to give compound **17** (912.4 mg, 3.2 mmol; yield: 80%) as a white solid. HRMS (ESI-TOF) [M+H]⁺ = 286.1316.

### 4-N,N-Dimethyl-5'-(O-4,4'-dimethoxytrityl)-2'-methoxycytidine (18)

After compound **17** (541.8 mg, 1.90 mmol) was dissolved in 6.5 mL of dry pyridine, 4,4'-dimethoxytrityl chloride (0.97 g, 2.90 mmol) was added. The reaction mixture was stirred at room temperature overnight followed by dilution with 40 mL of dichloromethane. The organic phase was serially washed with 5% aqueous Na₂S₂O₃ solution (2 × 20 mL), saturated aqueous sodium carbonate solution (20 mL), and saturated brine (20 mL). The organic phase was collected, dried over anhydrous sodium sulfate, and concentrated to give a crude product, which was separated by column chromatography to give compound **18** (745.8 mg, 1.27 mmol; yield: 67%) as a white solid. HRMS (ESI-TOF) [M+H]⁺ = 588.2651.

### 4-N,N-Dimethyl-5'-(O-4,4'-dimethoxytrityl)-2'-methoxycytidine, 3'-[(2-cyanoethyl)-(N,N-diisopropyl)]-phosphoramidite (19)

After compound **18** (469.8 mg, 0.8 mmol) was dissolved in 15 mL of dry dichloromethane, *N,N-*diisopropylethylamine (0.40 mL, 3.2 mmol) and 2-cyanoethyl-*N,N*-diisopropylphosphoramidite chloride (0.24 mL, 1.60 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **19** (440.9 mg, 0.56 mmol; yield: 70%) as a white solid. Molecular formula: C₄₂H₅₄N₅O₈P; HRMS (ESI-TOF) [M+H]⁺ = 788.3689.

### 1.5 Synthesis of 4-N-cyclopropyl-5'-(O-4,4'-dimethoxytrityl)-2'-fluoro-2'-deoxycytidine, 3'-[(2-cyanoethyl)-(N,N-diisopropyl)]-phosphoramidite (23)

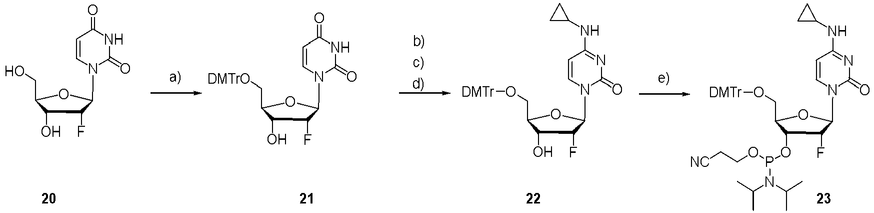

### 5'-(O-4,4'-dimethoxytrityl)-2'-fluoro-2'-deoxycytidine (21)

After compound **20** (5.0 g, 20.31 mmol) was dissolved in 75 mL of dry pyridine, 4,4'-dimethoxytrityl chloride (10.32 g, 30.46 mmol) was added. The reaction mixture was stirred at room temperature overnight and then separated by column chromatography to give compound **21** (6.68 g, 1.25 mmol; yield: 60%) as a white solid. HRMS (ESI-TOF) [M+H]⁺ = 549.2234.

### 4-N-cyclopropyl-5'-(O-4,4'-dimethoxytrityl)-5'-(O-4,4'-dimethoxytrityl)-2'-fluoro-2'-deoxycytidine (22)

After compound **21** (4.0 g, 7.29 mmol) was dissolved in 40 mL of dry pyridine, trimethylchlorosilane (TMSCl) (1.84 mL, 14.58 mmol) was added. The reaction mixture was stirred at room temperature for 2 hours and then labeled as solution A for later use. In another reaction flask, 40 mL of dry dichloromethane was sequentially added each time to a total of 200 mL for dilution. The organic phase was serially washed with 5% aqueous Na₂S₂O₃ solution (2 × 40 mL), saturated aqueous sodium carbonate solution (40 mL), and saturated brine (40 mL). The organic phase was collected, dried over anhydrous sodium sulfate, and concentrated to give a crude product; pyridine, triethylamine (1.39 mL, 72.9 mmol), and 1,2,4-triazole (1.51 g, 21.87 mmol) was added, followed by the addition of phosphorus oxychloride (3.35 g, 21.87 mmol). The reaction mixture was stirred at room temperature for 2 hours and then labeled as solution B. Solution A and solution B were homogeneously mixed and successively stirred for 4 hours, followed by the addition of cyclopropanamine (2.52 mL, 36.45 mmol). The mixture was successively stirred for 16 h and diluted with 200 mL of dichloromethane. The organic phase was serially washed with saturated aqueous sodium carbonate solution (40 mL) and saturated brine (40 mL). The organic phase was collected, dried over anhydrous sodium sulfate, and concentrated to give a crude product. After the resulting crude product was dissolved in 40 mL of tetrahydrofuran, tetrabutylammonium fluoride (TBAF) (14.58 mL, 1 M in THF) was added. The reaction mixture was stirred at room temperature for 16 hours and then concentrated to give a crude product, which was separated by column chromatography to give compound **22** (3.0 g, 5.1 mmol; yield: 70%) as a white solid. HRMS (ESI-TOF) [M+Na]⁺ = 609.2417.

### 4-N-cyclopropyl-5'-(O-4,4'-dimethoxytrityl)-2'-fluoro-2'-deoxycytidine, 3'-[(2-cyanoethyl)-(N,N-diisopropyl)]-phosphoramidite (23)

After compound **22** (1.0 g, 1.7 mmol) was dissolved in 10 mL of dry dichloromethane, *N,N-*diisopropylethylamine (0.89 mL, 5.1 mmol) and 2-cyanoethyl-*N,N*-diisopropylphosphoramidite chloride (0.57 mL, 2.55 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **23** (937.6 mg, 1.19 mmol; yield: 70%) as a white solid. HRMS (ESI-TOF) [M+H]⁺ = 788.3521.

### 1.6 Synthesis of 3-N-methyl-4-N-benzoyl-5'-(O-4,4'-dimethoxytrityl)-2'-methoxycytidine, 3'-[(2-cyanoethyl)-(N,N-diisopropyl)1-phosphoramidite (28)

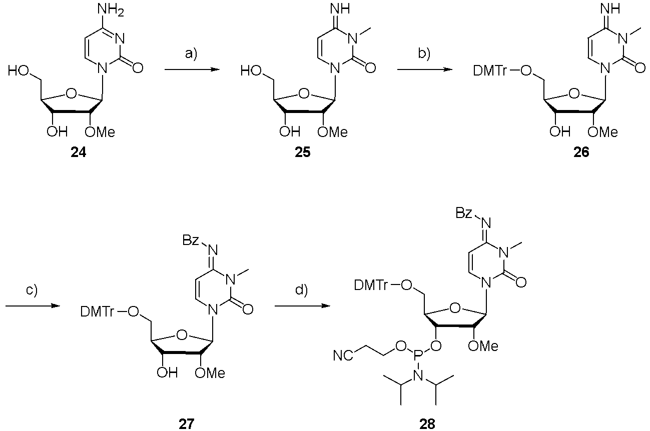

### 3-N-methyl-2'-methoxycytidine (25)

After 2'-methoxycytidine (**24**) (3.86 g, 15 mmol) was dissolved in dry DMF (40 mL), iodomethane (1.9 mL, 30.3 mmol) was added. The reaction solution was stirred at room temperature for 24 h before DMF was removed. The remaining crude product formed an azeotrope with toluene (2 × 80 mL) to remove the residual DMF and then reconstituted in 15 mL of acetone. *n*-Hexane (40 mL) was added, and the mixture was left to stand at -20 °C for 1 h. The resulting precipitate was filtered, and the filter cake was washed with cold acetone/*n*-hexane and dried under vacuum to give compound **25** (2.81 g, 10.4 mmol; yield: 69%) as a light yellow solid. Chemical Formula: C₁₁H₁₇N₃O₅; HRMS (ESI-TOF) [M+H]⁺ = 272.1151.

### 3-N-methyl-5'-(O-4,4'-dimethoxytrityl)-2'-methoxycytidine (26)

After compound **25** (2.5 g, 9.2 mmol) was dissolved in 30 mL of dry pyridine, 4,4'-dimethoxytrityl chloride (4.7 g, 14.0 mmol) was added. The reaction solution was stirred at room temperature overnight before 200 mL of dichloromethane was added. The organic phase was serially washed with 5% aqueous Na₂S₂O₃ solution (2 × 80 mL), saturated sodium carbonate (80 mL), and saturated brine (80 mL), then dried over anhydrous sodium sulfate, filtered, and concentrated under reduced pressure to remove the solvent. The remaining crude product was separated by column chromatography to give compound **26** (2.6 g, 4.6 mmol; yield: 50%) as a white solid. Molecular formula: C₃₂H₃₅N₃O₇; HRMS (ESI-TOF) [M+H]⁺ = 574.2511.

### 3-N-methyl-4-N-benzoyl-5'-(O-4,4'-dimethoxytrityl)-2'-methoxycytidine (27)

Compound **26** (2.29 g, 4.0 mmol) was distilled under reduced pressure with pyridine (2 × 50 mL) to remove excessive water, and then reconstituted in 50 mL of dry pyridine. After trimethylsilyl chloride (TMSCl) (2.1 mL, 16.4 mmol) was added, the mixture was stirred at room temperature for 1.5 hours. Benzoyl chloride (BzCl; 0.69 g, 4.92 mmol) was added, and the mixture was successively stirred at room temperature for 4 hours. After the reaction was complete, 10 mL of water was added, and the mixture was stirred at room temperature for 5 min, followed by the addition of aqueous ammonia (15 mL, 25-28% w/w). The reaction mixture was successively stirred at room temperature for 15 min followed by distillation under reduced pressure to remove the solvent. The remaining crude product was separated by column chromatography to give compound **27** (2.0 g, 2.95 mmol; yield: 74%) as a white solid. Molecular formula: C₃₉H₃₉N₃O₈; HRMS (ESI-TOF) [M+H]⁺ = 678.2729.

### 3-N-methyl-4-N-benzoyl-5'-(O-4,4'-dimethoxytrityl)-2'-methoxycytidine, -3'-[(2-cyanoethyl)-(N,N-diisopropyl)]-phosphoramidite (28)

After compound **27** (541.8 mg, 0.8 mmol) was dissolved in 15 mL of dry dichloromethane, *N,N-*diisopropylethylamine (0.40 mL, 3.2 mmol) and 2-cyanoethyl-*N,N*-diisopropylphosphoramidite chloride (0.24 mL, 1.60 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **28** (422.3 mg, 0.48 mmol; yield: 60%) as a white solid. Molecular formula: C₄₈H₅₆N₅O₉P; HRMS (ESI-TOF) [M+H]⁺ = 878.3851.

### 1.7 Synthesis of 4-N-methyl-5'-(O-4,4'-dimethoxytrityl)-2'-methoxycytidine, 3'-[(2-cyanoethyl)-(N,N-diisopropyl)]-phosphoramidite (32)

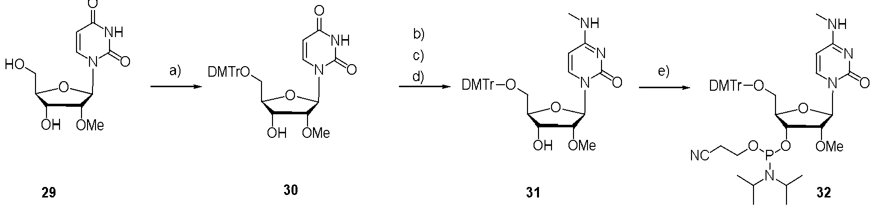

### 5'-(O-4,4'-dimethoxytrityl)-2'-methoxyuridine (30)

After compound **29** (5.2 g, 20.1 mmol) was dissolved in 75 mL of dry pyridine, 4,4'-dimethoxytrityl chloride (10.32 g, 30.5 mmol) was added. The reaction mixture was stirred at room temperature overnight and then separated by column chromatography to give compound **30** (7.01 g, 12.5 mmol; yield: 62%) as a white solid. Molecular formula: C₃₁H₃₂N₂O₈; HRMS (ESI-TOF) [M+H]⁺ = 561.2165.

### 4-N-methyl-5'-(O-4,4'-dimethoxytrityl)-2'-methoxycytidine (31)

After compound **30** (4.0 g, 7.21 mmol) was dissolved in 40 mL of dry pyridine, trimethylchlorosilane (TMSCl) (1.84 mL, 14.58 mmol) was added. The reaction mixture was stirred at room temperature for 2 hours and then labeled as solution A for later use. In another reaction flask, 40 mL of dry dichloromethane was sequentially added each time to a total of 200 mL for dilution. The organic phase was serially washed with 5% aqueous Na₂S₂O₃ solution (2 × 40 mL), saturated aqueous sodium carbonate solution (40 mL), and saturated brine (40 mL). The organic phase was collected, dried over anhydrous sodium sulfate, and concentrated to give a crude product; pyridine, triethylamine (1.39 mL, 72.9 mmol), and 1,2,4-triazole (1.51 g, 21.87 mmol) was added, followed by the addition of phosphorus oxychloride (3.35 g, 21.87 mmol). The reaction mixture was stirred at room temperature for 2 hours and then labeled as solution B. Solution A and solution B were homogeneously mixed and successively stirred for 4 hours, followed by the addition of methylamine solution. The mixture was successively stirred for 16 hours and then diluted with 200 mL of dichloromethane. The organic phase was serially washed with saturated aqueous sodium carbonate solution (40 mL) and saturated brine (40 mL). The organic phase was collected, dried over anhydrous sodium sulfate, and concentrated to give a crude product. After the resulting crude product was dissolved in 40 mL of tetrahydrofuran, tetrabutylammonium fluoride (TBAF) (14.58 mL, 1 M in THF) was added. The reaction mixture was stirred at room temperature for 16 hours and then concentrated to give a crude product, which was separated by column chromatography to give compound **31** (3.0 g, 5.2 mmol; yield: 73%) as a white solid. Molecular formula: C₃₂H₃₅N₃O₇; HRMS (ESI-TOF) [M+Na]⁺ = 597.2384.

### 4-N-methyl-5'-(O-4,4'-dimethoxytrityl)-2'-methoxycytidine, 3'-[(2-cyanoethyl)-(N,N-diisopropyl)]-phosphoramidite (32)

After compound **31** (975.2 mg, 1.7 mmol) was dissolved in 10 mL of dry dichloromethane, *N,N-*diisopropylethylamine (0.89 mL, 5.1 mmol) and 2-cyanoethyl-*N,N*-diisopropylphosphoramidite chloride (0.57 mL, 2.55 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **32** (727.4 mg, 0.94 mmol; yield: 55%) as a white solid. Molecular formula: C₄₁H₅₂N₅O₈P; HRMS (ESI-TOF) [M+H]⁺ = 774.3566.

### 1.8 Synthesis of 4-N-cyclopropyl-5'-(O-4,4'-dimethoxytrityl)-2'-methoxycytidine, 3'-[(2-cyanoethyl)-(N,N-diisopropyl)]-phosphoramidite (34)

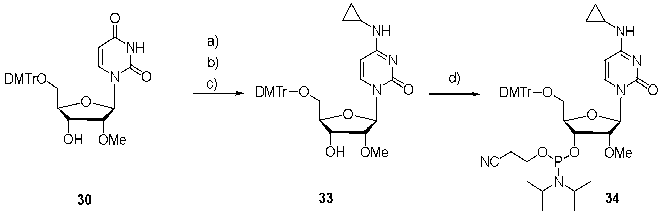

### 4-N-cyclopropyl-5'-(O-4,4'-dimethoxytrityl)-2'-methoxycytidine (33)

After compound **30** (4.1 g, 7.30 mmol) was dissolved in 40 mL of dry pyridine, trimethylchlorosilane (TMSCl) (1.84 mL, 14.58 mmol) was added. The reaction mixture was stirred at room temperature for 2 hours and then labeled as solution A for later use. In another reaction flask, 40 mL of dry dichloromethane was sequentially added each time to a total of 200 mL for dilution. The organic phase was serially washed with 5% aqueous Na₂S₂O₃ solution (2 × 40 mL), saturated aqueous sodium carbonate solution (40 mL), and saturated brine (40 mL). The organic phase was collected, dried over anhydrous sodium sulfate, and concentrated to give a crude product; pyridine, triethylamine (1.39 mL, 72.9 mmol), and 1,2,4-triazole (1.51 g, 21.87 mmol) was added, followed by the addition of phosphorus oxychloride (3.35 g, 21.87 mmol). The reaction mixture was stirred at room temperature for 2 hours and then labeled as solution B. Solution A and solution B were homogeneously mixed and successively stirred for 4 hours, followed by the addition of cyclopropanamine (2.52 mL, 36.45 mmol). The mixture was successively stirred for 16 h and diluted with 200 mL of dichloromethane. The organic phase was serially washed with saturated aqueous sodium carbonate solution (40 mL) and saturated brine (40 mL). The organic phase was collected, dried over anhydrous sodium sulfate, and concentrated to give a crude product. After the resulting crude product was dissolved in 40 mL of tetrahydrofuran, tetrabutylammonium fluoride (TBAF) (14.58 mL, 1 M in THF) was added. The reaction mixture was stirred at room temperature for 16 hours and then concentrated to give a crude product, which was separated by column chromatography to give compound **33** (2.5 g, 4.2 mmol; yield: 60%) as a white solid. Molecular formula: C₃₄H₃₇N₃O₇; HRMS (ESI-TOF) [M+H]⁺ = 600.2639.

### 4-N-cyclopropyl-5'-(O-4,4'-dimethoxytrityl)-2'-methoxycytidine, 3'-[(2-cyanoethyl)-(N,N-diisopropyl)]-phosphoramidite (34)

After compound **33** (1.0 g, 1.7 mmol) was dissolved in 10 mL of dry dichloromethane, *N,N-*diisopropylethylamine (0.89 mL, 5.1 mmol) and 2-cyanoethyl-*N,N*-diisopropylphosphoramidite chloride (0.57 mL, 2.55 mmol) were added. The reaction solution was stirred at room temperature overnight under nitrogen atmosphere, quenched with the addition of water, and then extracted with ethyl acetate. The organic phase was collected, dried over anhydrous sodium sulfate, and concentrated under reduced pressure to give a crude product, which was separated by column chromatography to give compound **34** (879.9 mg, 1.10 mmol; yield: 65%) as a white solid. Molecular formula: C₄₃H₅₄N₅O₈P; HRMS (ESI-TOF) [M+H]⁺ = 800.3727.

**Table 1: Modified nucleoside monomers used in the present disclosure**

| **Monomer name** | **2M4Cm** | **2M4Cf** | **Cp4Cm** | **CP4Cf** |
|---|---|---|---|---|
| Specific structure of the monomer in the oligonucleotide | 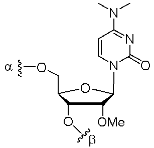 | 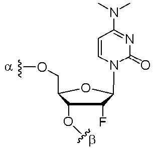 | 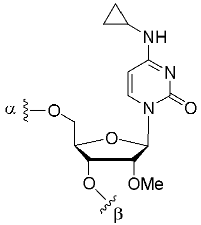 | 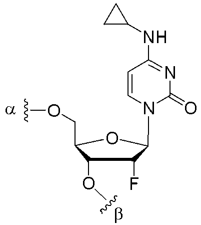 |
| Structural formula of the monomer in the form of protective group-bearing | 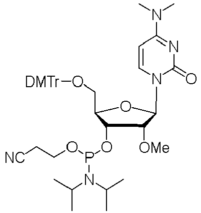 | 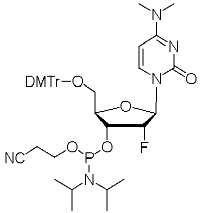 | 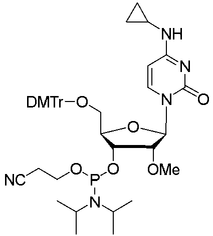 | 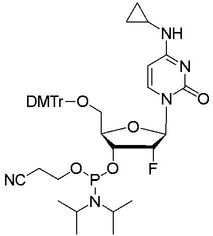 |

| **Monomer name** | **M3Cm** | **M3Cf** | **M4Cm** | **M4Cf** |
|---|---|---|---|---|
| Specific structure of the monomer in the oligonucleotide | 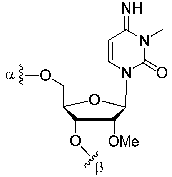 | 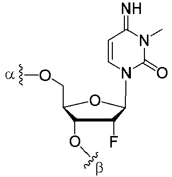 | 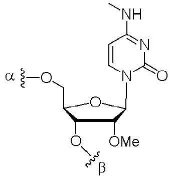 | 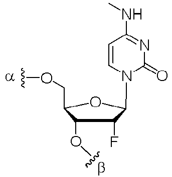 |
| Structural formula of the monomer in the form of protective group-bearing | 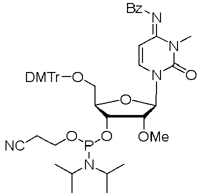 | 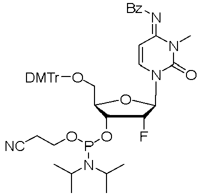 | 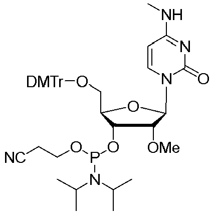 | 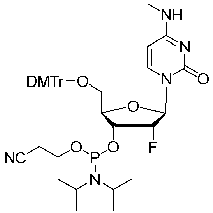 |

| | | | | |
|---|---|---|---|---|
| Note: Relevant protective groups need to be borne by the relevant monomers of the present disclosure prior to dsRNA synthesis and removed during dsRNA synthesis to form the specific structures in the oligonucleotides. | | | | |

### Example 2: Synthesis and Purification of Oligonucleotides

All oligonucleotides were synthesized on an LK-192X synthesizer using a 500 nmol universal Frit support (1000 Å = 100 nm). All phosphoramidite monomers were diluted with an anhydrous acetonitrile solvent in a ratio of 1:40 (g/mL) and subjected to a total of two couplings with a coupling duration of 3 min. Deprotection was performed with 3% TCA; activation was performed with 0.3 M benzylthiotetrazole solution in acetonitrile; capping and oxidation were performed with CAPA/CAPB and 50 mM I₂ solution, respectively. After the trityl-off synthesis, the solid phase support was transferred to a 2 mL centrifuge tube; 1.2 mL of aqueous ammonia was added, and the mixture was heated in an oven at 65 °C for 3 h to remove the protective group. The mixture was then cooled to room temperature and concentrated under vacuum for 30 min. The solution was filtered through a 0.22 µm filter membrane into a sample vial to perform single strand purification using a semi-preparative reversed-phase purification instrument, the elution gradient being 7%-30% (ACN:100 mM TEAA), the time being 10 min, and the flow rate being 5 mL/min. After the purification and preparation, the solution was concentrated under vacuum and concentrated by rotary evaporation at room temperature. The samples were finally dissolved in water and each solution was desalted on a GE Hi-Trap desalting column to elute the final oligonucleotide product. All identities and purities were confirmed using ESI-MS and IEX HPLC, respectively. Ultraviolet concentration determination was performed using a microplate reader, and an equimolar amount of the sense strand and the antisense strand were mixed in a new reaction tube. The mixture was heated at 95 °C for 5 min, slowly annealed to room temperature, and finally concentrated by rotary evaporation at room temperature using a vacuum concentrator to give the final product.

**Table 2: Synthesized oligonucleotides**

| siRNA sequence | SS strand 5'-3' | AS strand 5'-3' | Target |
|---|---|---|---|
| NC | UUCUCCGAACGUGUCACGUTT(Seq No:43) | ACGUGACACGUUCGGAGAATT(Seq No:44) | Scramble |
| PC-1 | AfsAmsUfGmAfCmUfUmUfUfAfUmUfGmAfGmCfUmCfUmUf(Se q No:22) | AmsAfsGmAfGmCfUmCfAmAfUmAmAmAfAmGfUmCfAmUfUmsCmsUm(Seq No:45) | PCSK9 |
| PMC-1 | AfsAmsUfGmAfCmUfUmUfUfAfUmUfGmAfGmCfUmCfUmUf | AmsAfsGmAfGm/**M3Cf**/UmCfAmAfUmAmAmAfAmGfUmCfAmUfUmsCmsUm(Seq No:46) | PCSK9 |
| PMC-2 | AfsAmsUfGmAfCmUfUmUfUfAfUmUfGmAfGmCfUmCfUmUf | AmsAfsGmAfGmCfUm/**M3Cf**/AmAfUmAmAmAfAmGfUmCfAmUfUmsCmsUm(Seq No:47) | PCSK9 |
| PMC-3 | AfsAmsUfGmAfCmUfUmUfUfAfUmUfGmAfGmCfUmCfUmUf | AmsAfsGmAfGm/**2M4Cf**/UmCfAmAfUmAmAmAfAmGfUmCfAmUfUmsCmsUm(Seq No:48) | PCSK9 |
| PMC-4 | AfsAmsUfGmAfCmUfUmUfUfAfUmUfGmAfGmCfUmCfUmUf | AmsAfsGmAfGm/**M4Cf**/UmCfAmAfUmAmAmAfAmGfUmCfAmUfUmsCmsUm(Seq No:49) | PCSK9 |
| PMC-5 | AfsAmsUfGmAfCmUfUmUfUfAfUmUfGmAfGmCfUmCfUmUf | AmsAfsGmAfGmCfUm/**M4Cf**/AmAfUmAmAmAfAmGfUmCfAmUfUmsCmsUm(Seq No:50) | PCSK9 |
| PMC-6 | AfsAmsUfGmAfCmUfUmUfUfAfUmUfGmAfGmCfUmCfUmUf | AmsAfsGmAfGm/**CP4Cf**/UmCfAmAfUmAmAmAfAmGfUmCfAmUfUmsCmsUm(Seq No:51) | PCSK9 |
| PMC-7 | AfsAmsUfGmAfCmUfUmUfUfAfUmUfGmAfGmCfUmCfUmUf | AmsAfsGmAfGmCfUm/**cp4Cf**/AmAfUmAmAmAfAmGfUmCfAmUfUmsCmsUm(Seq No:52) | PCSK9 |
| PC-2 | CmsCmsAmCmAmAmCfGmCfUfUfUmUmGmGmGmGmGmUmG mAm(Seq No:25) | UmsCfsAmCmCmCfCmCmAmAmAmAmGmCfGmUfUmGmUmGmGmsGmsCm(Seq No:53) | PCSK9 |
| PMC-8 | CmsCmsAmCmAmAmCfGmCfUfUfUmUmGmGmGmGmGmUmG mAm | UmsCfsAmCmCm/**2M4Cf**/CmCmAmAmAmAmGmCfGmUfUmGmUmGmGmsGmsCm(Seq No:54) | PCSK9 |
| PMC-9 | CmsCmsAmCmAmAmCfGmCfUfUfUmUmGmGmGmGmGmUmG mAm | UmsCfsAmCmCmCf/**2M4Cf**/CmAmAmAmAmGmCfGmUfUmGmUmGmGmsGmsCm(Seq No:55) | PCSK9 |
| PMC-10 | CmsCmsAmCmAmAmCfGmCfUfUfUmUmGmGmGmGmGmUmG mAm | UmsCfsAmCmCm/**CP4Cf**/CmCmAmAmAmAmGmCfGmUfUmGmUmGmGmsGmsCm(Seq No:56) | PCSK9 |
| PMC-11 | CmsCmsAmCmAmAmCfGmCfUfUfUmUmGmGmGmGmGmUmG mAm | UmsCfsAmCmCmCf/**CP4Cf**/CmAmAmAmAmGmCfGmUfUmGmUmGmGmsGmsCm(Seq No:57) | PCSK9 |
| PMC-12 | CmsCmsAmCmAmAmCfGmCfUfUfUmUmGmGmGmGmGmUmG mAm | UmsCfsAmCmCmCfCm/**CP4Cf**/AmAmAmAmGmCfGmUfUmGmUmGmGmsGmsCm(Seq No:58) | PCSK9 |
| PMC-13 | CmsCmsAmCmAmAmCfGmCfUfUfUmUmGmGmGmGmGmUmG mAm | UmsCfsAmCmCm/**2M4Cm**/CmCmAmAmAmAmGmCfGmUfUmGmUmGmGmsGmsCm(Seq No:59) | PCSK9 |
| PMC-14 | CmsCmsAmCmAmAmCfGmCfUfUfUmUmGmGmGmGmGmUmG mAm | UmsCfsAmCmCmCf/**2M4Cm**/CmAmAmAmAmGmCfGmUfUmGmUmGmGmsGmsCm(Seq No:60) | PCSK9 |
| PC-3 | GmsAmsGmAmAmUmUfUmUfGfGfUmUmGmGmGmCmCmUmA mGm(Seq No:29) | CmsUfsAmGmGmCfCmCmAmAmCmCmAmAfAmAfUmUmCmUmCmsCmsAm(Seq No:61) | ANGPTL3 |
| AMC-1 | GmsAmsGmAmAmUmUfUmUfGfGfUmUmGmGmGmCmCmUmA mGm | CmsUfsAmGmGm/**2M4Cf**/CmCmAmAmCmCmAmAfAmAflJmUmCmUmCmsCmsAm(Seq No:62) | ANGPTL3 |
| AMC-2 | GmsAmsGmAmAmUmUfUmUfGfGfUmUmGmGmGmCmCmUmA mGm | CmsUfsAmGmGmCf/**2M4Cf**/CmAmAmCmCmAmAfAmAfUmUmCmUmCmsCmsAm(Seq No:63) | ANGPTL3 |
| AMC-3 | GmsAmsGmAmAmUmUfUmUfGfGfUmUmGmGmGmCmCmUmA mGm | CmsUfsAmGmGm/**CP4Cf**/CmCmAmAmCmCmAmAfAmAfUmUmCmUmCmsCmsAm(Seq No:64) | ANGPTL3 |
| AMC-4 | GmsAmsGmAmAmUmUfUmUfGfGfUmUmGmGmGmCmCmUmA mGm | CmsUfsAmGmGmCf/**CP4Cf**/CmAmAmCmCmAmAfAmAfUmUmCmUmCmsCmsAm(Seq No:65) | ANGPTL3 |
| AMC-5 | GmsAmsGmAmAmUmUfUmUfGfGfUmUmGmGmGmCmCmUmA mGm | CmsUfsAmGmGmCfCm/**CP4Cf**/AmAmCmCmAmAfAmAfUmUmCmUmCmsCmsAm(Seq No:66) | ANGPTL3 |
| PC-4 | CmsCmsCmAmGmUmGfUmCfAfGfGmUmGmGmGmAmGmUmA mCm(Seq No:33) | GmsUfsAmCmUmCfCmCmAmCmCmUmGmAfCmAfCmUmGmGmGmsAmsUm(Seq No:67) | LPA |
| LMC-1 | CmsCmsCmAmGmUmGfUmCfAfGfGmUmGmGmGmAmGmUmA mCm | GmsUfsAmCmUm/**2M4Cf**/CmCmAmCmCmUmGmAfCmAfCmUmGmGmGmsAmsUm(Seq No:68) | LPA |
| LMC-2 | CmsCmsCmAmGmUmGfUmCfAfGfGmUmGmGmGmAmGmUmA mCm | GmsUfsAmCmUm/**CP4Cf**/CmCmAmCmCmUmGmAfCmAfCmUmGmGmGmsAmsUm(Seq No:69) | LPA |
| LMC-3 | CmsCmsCmAmGmUmGfUmCfAfGfGmUmGmGmGmAmGmUmA mCm | GmsUfsAmCmUmCf/**CP4Cf**/CmAmCmCmUmGmAfCmAfCmUmGmGmGmsAmsUm(Seq No:70) | LPA |
| LMC-4 | CmsCmsCmAmGmUmGfUmCfAfGfGmUmGmGmGmAmGmUmA mCm | GmsUfsAmCmUmCfCm/**CP4Cf**/AmCmCmUmGmAfCmAfCmUmGmGmGmsAmsUm(Seq No:71) | LPA |

| | | | |
|---|---|---|---|
| Note: SS: sense strand; AS: antisense strand; replaced bases are shown in bold in specific sequences. Scramble RNA is a scrambled control, which means that the bases of a control sequence are the same as those of an RNAi target sequence, but the bases of the target sequence are scrambled. | | | |

**Table 3. Mass spectrometry characterization data for synthesized sequences**

| **siRNA sequence** | **SS strand theoretical molecular weight** | **SS: sense strand mass** | **AS strand theoretical molecular weight** | **AS: antisense strand mass** |
|---|---|---|---|---|
| PC-1 | 6778.96 | 6779.5 | 7601.60 | 7602.8 |
| NC | 7373.61 | 7374.1 | 6732.19 | 6732.7 |
| PMC-1 | 6778.96 | 6777.1 | 7615.60 | 7617.4 |
| PMC-2 | 6778.96 | 6777.6 | 7615.60 | 7617.2 |
| PMC-3 | 6778.96 | 6779.0 | 7629.56 | 7629.3 |
| PMC-4 | 6778.96 | 6780.3 | 7615.53 | 7615.2 |
| PMC-5 | 6778.96 | 6779.5 | 7615.53 | 7615.1 |
| PMC-6 | 6778.96 | 6780.0 | 7643.41 | 7642.4 |
| PMC-7 | 6778.96 | 6780.2 | 7643.42 | 7642.5 |
| PC-2 | 7006.10 | 7005.4 | 7659.47 | 7660.0 |
| PMC-8 | 7006.10 | 7006.3 | 7689.37 | 7687.7 |
| PMC-9 | 7006.10 | 7006.3 | 7677.37 | 7675.5 |
| PMC-10 | 7006.10 | 7006.4 | 7701.37 | 7700.4 |
| PMC-11 | 7006.10 | 7006.8 | 7689.37 | 7688.9 |
| PMC-12 | 7006.10 | 7006.6 | 7689.37 | 7687.5 |
| PMC-13 | 7006.10 | 7005.5 | 7701.39 | 7700.3 |
| PMC-14 | 7006.10 | 7005.3 | 7689.39 | 7688.1 |
| PC-3 | 7048.11 | 7049.2 | 7571.44 | 7571.3 |
| AMC-1 | 7048.11 | 7049.6 | 7601.34 | 7599.3 |
| AMC-2 | 7048.11 | 7049.1 | 7589.34 | 7587.0 |
| AMC-3 | 7048.11 | 7049.0 | 7613.34 | 7611.3 |
| AMC-4 | 7048.11 | 7048.8 | 7601.34 | 7599.1 |
| AMC-5 | 7048.11 | 7048.9 | 7601.34 | 7599.1 |
| PC-4 | 7045.14 | 7046.2 | 7620.43 | 7620.4 |
| LMC-1 | 7045.14 | 7046.5 | 7650.33 | 7648.0 |
| LMC-2 | 7045.14 | 7047.3 | 7662.33 | 7659.9 |
| LMC-3 | 7045.14 | 7046.6 | 7650.33 | 7648.9 |
| LMC-4 | 7045.14 | 7046.3 | 7650.33 | 7648.5 |

### Example 3: Anti-Off-Target Evaluation Experiment on RNA Sequences

The cells transfected with the dsRNAs of the present disclosure were sequenced by an RNA-seq technique to obtain the expression pattern of whole mRNAs, and the data were analyzed by a biological information analysis method to obtain the gene regulation data of the dsRNAs described above. The brief steps are as follows:
Hep3B or RT4 cells were separately cultured in a DMEM culture medium containing 10% FBS (1.5 mg/L Glutamine, 100 U/mL Penicillin, 100 µg/mL Streptomycin) or a MCCOY'S 5A culture medium containing 10% FBS (100 U/mL Penicillin, 100 µg/mL Streptomycin) in an incubator at 37 °C with 5% CO₂ and saturated humidity. The cells were transfected with 10 nM (PCSK9/ANGPTL3) or 100 nM (LPA) dsRNAs in Table 4 using 2 µg/mL Lipo2000 (Invitrogen) in a 12-well plate (200,000 cells/well). The cells were harvested 48 hours after transfection, and RNA extraction was performed using a TRIZOL method. A cDNA library was prepared using a TruSeq Stranded Total RNA Library Prep kit (Illumina), and sequencing was performed on the DNBSEQ platform. The original RNAseq reads were filtered using an SOAPnuke software from BGI genomics, and the specific steps are as follows: 1) removing reads containing a linker (linker contamination); 2) removing reads with unknown base N content greater than 5%; 3) removing low-quality reads (reads having a ratio of bases with quality values less than 15 to the total number of bases in the reads greater than 20% were defined as low-quality reads). Finally, the filtered reads were aligned with the human genome (GCF_000001405.39_GRCh38.p13) using HISAT (RNA-seq calibrator), and unique alignment reads were calculated by featureCounts. Differential gene expression analysis was performed by R package DESeq2. The results were as follows:

**Table 4: Results of off-target effect related to up-regulation and down-regulation of gene expression caused by dsRNAs**

| siRNA sequence | Target | Control | AS strand modification site | Modified monomer | Number of down-regulated genes | Number of up-regulated genes | Total | Off-target effects |
|---|---|---|---|---|---|---|---|---|
| PC-1 | PCSK9 | VS NC | / | / | 66 | 90 | 156 | / |
| PMC-1 | PCSK9 | VS NC | Site 6 | M3Cf | 39 | 19 | 58 | -63% |
| PMC-2 | PCSK9 | VS NC | Site 8 | M3Cf | 69 | 77 | 146 | -6% |
| PMC-3 | PCSK9 | VS NC | Site 6 | 2M4Cf | 21 | 9 | 30 | -81% |
| PMC-4 | PCSK9 | VS NC | Site 6 | M4Cf | 24 | 26 | 50 | -68% |
| PMC-5 | PCSK9 | VS NC | Site 8 | M4Cf | 63 | 62 | 125 | -20% |
| PMC-6 | PCSK9 | VS NC | Site 6 | CP4Cf | 89 | 53 | 142 | -9% |
| PMC-7 | PCSK9 | VS NC | Site 8 | CP4Cf | 22 | 29 | 51 | -67% |
| PC-2 | PCSK9 | VS blank | / | / | 59 | 137 | 196 | / |
| PMC-8 | PCSK9 | VS blank | Site 6 | 2M4Cf | 110 | 36 | 146 | -26% |
| PMC-13 | PCSK9 | VS blank | Site 6 | 2M4Cm | 52 | 37 | 89 | -55% |
| PC-3 | ANGPTL3 | VS blank | / | / | 11 | 42 | 53 | / |
| AMC-1 | ANGPTL3 | VS blank | Site 6 | 2M4Cf | 4 | 7 | 11 | -79% |
| AMC-4 | ANGPTL3 | VS blank | Site 7 | CP4Cf | 9 | 26 | 35 | -34% |
| PC-4 | LPA | VS blank | / | / | 52 | 34 | 86 | / |
| LMC-1 | LPA | VS blank | Site 6 | 2M4Cf | 28 | 53 | 81 | -6% |

| | | | | | | | | |
|---|---|---|---|---|---|---|---|---|
| Note: In the above table, VS NC represents the change of genes by the target sequence relative to the negative control sequence (NC); VS blank represents the change of genes by the target sequence relative to the blank control sequence (blank), in which UP represents the up-regulated genes; Down represents the down-regulated genes; and the sum of the two is the number of gene expressions possibly affected by the sequence in the statistical model; the higher the number, the more affected genes are represented, i.e., the greater the off-target possibility and extent are. | | | | | | | | |

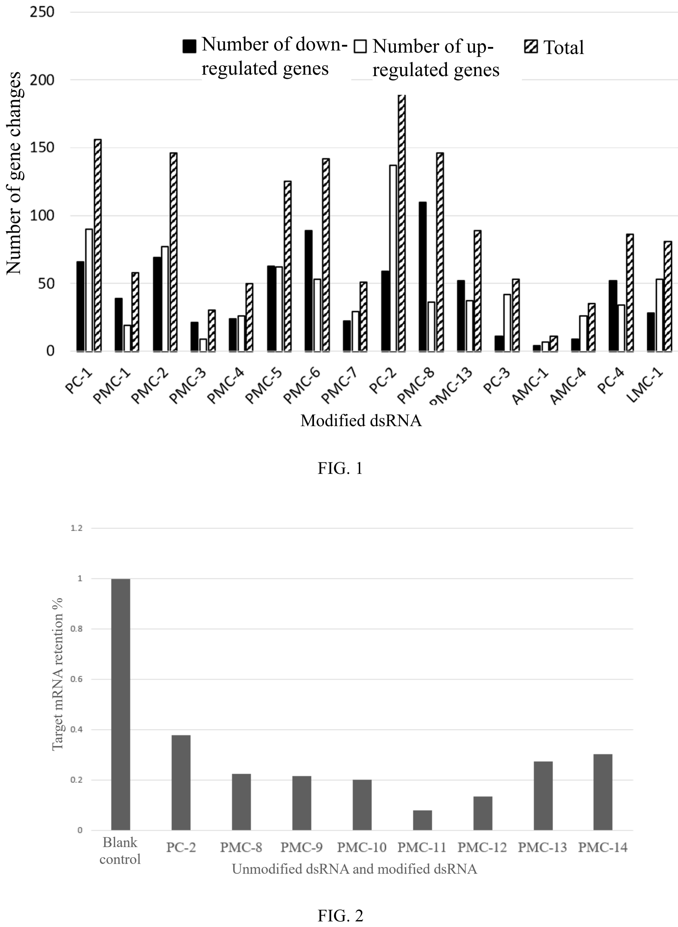

According to the experimental effect data in Table 4 and FIG. 1, the incorporation of the modified cytidine of the present disclosure into sites 6-8 of the dsRNA AS strands exhibited a satisfactory reduction in off-target effects, and this result repeated itself in a variety of different sequences of different targets, indicating that the reduction in off-target effects by the modified cytidine of the present disclosure at sites 6-8 of the AS strands had predictable universality. In addition, the results demonstrate that different modifications on the sugar ring had little effect on the off-target effect. For example, the anti-off-target effect data of the 2'-F modification and the 2'-Ome modification 2M4C are only slightly different, and both exhibited anti-off-target effects with the same trend in the same sequence and at the same site.

### Example 4: Efficacy Evaluation Experiment of RNA Drug Sequences

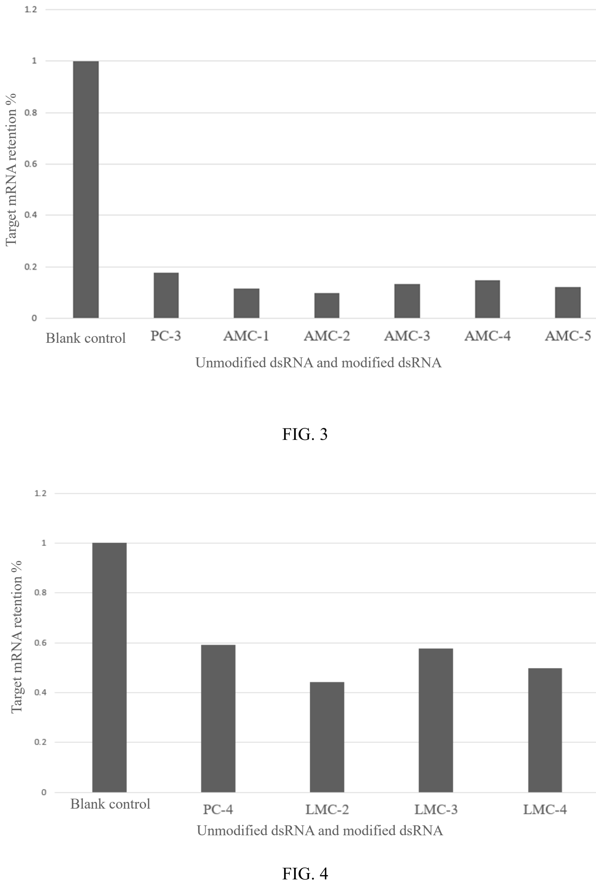

Hep3B or RT4 cells were separately cultured in a DMEM culture medium containing 10% FBS (1.5 mg/L Glutamine, 100 U/mL Penicillin, 100 µg/mL Streptomycin) or a MCCOY'S 5A culture medium containing 10% FBS (100 U/mL Penicillin, 100 µg/mL Streptomycin) in an incubator at 37 °C with 5% CO₂ and saturated humidity. The cells were transfected with 10 nM (PCSK9/ANGPTL3) or 100 nM (LPA) dsRNAs using 2 µg/mL Liposome 2000 (Thermo Fisher Scientific) in a 24-well plate (100,000 cells/well). Total cellular RNA was extracted from the cell sample according to the instructions of the RNA extraction kit and quantified using NanoDrop. The target genes and GAPDH gene in the total RNA of the cells were assayed by the duplex RT-qPCR assay. The relative expression level of the target genes in cells of other experimental groups was analyzed by a relatively quantified analytical method using a reference gene as a standard, wherein the GADPH gene was the reference gene, and the blank was the control. After the cells were transfected with the sequences described above, the modified dsRNAs of the present disclosure and the unmodified PC groups were compared in terms of the knockout assay of the target genes. The results are shown in Table 5 and FIGs. 2-4:

**Table 5. Results of comparative experiment for effect of dsRNAs on knockdown activity**

| **siRNA sequence** | **Target** | **AS strand modification site** | **Modified monomer** | **Change in knockdown activity relative to the PC groups** |
|---|---|---|---|---|
| **PC-2** | **PCSK9** | / | / | / |
| PMC-8 | PCSK9 | Site 6 | 2M4Cf | +40% |
| PMC-9 | PCSK9 | Site 7 | 2M4Cf | +42% |
| PMC-10 | PCSK9 | Site 6 | CP4Cf | +46% |
| PMC-11 | PCSK9 | Site 7 | CP4Cf | +79% |
| PMC-12 | PCSK9 | Site 8 | CP4Cf | +65% |
| PMC-13 | PCSK9 | Site 6 | 2M4Cm | +27% |
| PMC-14 | PCSK9 | Site 7 | 2M4Cm | +20% |
| PC-3 | ANGPTL3 | / | / | / |
| AMC-1 | ANGPTL3 | Site 6 | 2M4Cf | +35% |
| AMC-2 | ANGPTL3 | Site 7 | 2M4Cf | +44% |
| AMC-3 | ANGPTL3 | Site 6 | CP4Cf | +24% |
| AMC-4 | ANGPTL3 | Site 7 | CP4Cf | +16% |
| AMC-5 | ANGPTL3 | Site 8 | CP4Cf | +31% |
| PC-4 | LPA | / | / | / |
| LMC-2 | LPA | Site 6 | CP4Cf | +25% |
| LMC-3 | LPA | Site 7 | CP4Cf | +2% |
| LMC-4 | LPA | Site 8 | Cp4Cf | +16% |

The results demonstrate that the dsRNAs containing the modified nucleoside monomers of the present disclosure at sites 6-8 of the AS strands all were able to achieve the "silencing" effect on the target genes, and were able to significantly reduce the expression effect of the target genes compared with the negative controls (PC-2, PC-3, and PC-4) that did not contain the modified nucleoside monomers of the present disclosure. Moreover, the modified nucleoside monomers of the present disclosure all exhibited the effects described above in a variety of different sequences of different targets. The data described above indicate that the dsRNAs of the present disclosure had superior pharmacodynamic functions relative to the dsRNAs that did not contain the modified nucleoside monomer of the present disclosure.

### Example 5: Anti-Off-Target and Efficacy Evaluation Experiment for Targeting Complement C5 Gene

Furthermore, the anti-off-target effect was verified in dsRNAs that target the complement C5 gene and are related to GeneBank accession GI: 1732746243 and reference sequence: NM_001735.3. Table 6 is a list of sequences, in which the synthetic steps are the same as Example 2.

**Table 6: Synthesized oligonucleotides**

| siRNA sequence | SS strand 5'-3' | AS strand 5'-3' | Target |
|---|---|---|---|
| E5 | GmsUmsGmAmUmUmCfAmAfGfUfUmUmAm UmGmGmAmUmAmCm(Seq No:37) | GmsUfsAmUmCmCmAmUmAmAmAmCmUmUfG mAfAmUmCmAmCmsAmsAm(Seq No:72) | C5 |
| FE5 | GmsUmsGmAmUmUmCfAmAfGfUfUmUmAm UmGmGmAmUmAmCm | GmsUfsAmUmCm/**2M4Cm**/AmUmAmAmAmCmU mUfGmAfAmUmCmAmCmsAmsAm(Seq No:73) | |
| E17 | UmsGmsUmGmUmAmUfUmUfGfGfAmAmGm UmUmGmUmAmUmCm(Seq No:39) | GmsAfsUmAmCmAmAmCmUmUmCmCmAmAfAm UfAmCmAmCmAmsUmsAm(Seq No:74) | |
| FE17 | UmsGmsUmGmUmAmUfUmUfGfGfAmAmGm UmUmGmUmAmUmCm | GmsAfsUmAmCmAmAm/**2M4Cm**/UmUmCmCmA mAfAmUfAmCmAmCmAmsUmsAm(Seq No:75) | |
| E85 | GmsUmsGmAmAmGmAfAmAfUfGfUmUmGm UmUmAmCmGmAmUm(Seq No:41) | AmsUfsCmGmUmAmAmCmAmAmCmAmUmUfU mCfUmUmCmAmCmsUmsAm(Seq No:76) | |
| FE85 | GmsUmsGmAmAmGmAfAmAfUfGfUmUmGm UmUmAmCmGmAmUm | AmsUfsCmGmUmAmAm/**2M4Cm**/AmAmCmAmU mUfUmCfUmUmCmAmCmsUmsAm(Seq No:77) | |

| | | | |
|---|---|---|---|
| Note: SS: sense strand; AS: antisense strand; replaced bases are shown in bold in specific sequences. | | | |

The cells transfected with the dsRNAs of the present disclosure were sequenced by an RNA-seq technique to obtain the expression pattern of whole mRNAs, and the data were analyzed by a biological information analysis method to obtain the gene regulation data of the dsRNAs described above. The brief steps are as follows:
HepG2 cells were cultured in a DMEM culture medium containing 10% fetal bovine serum in an incubator at 37 °C with 5% CO₂. The cells were digested, then adjusted to a cell density of 2 × 10⁵ cells/mL, and seeded into a 24-well plate at 1 mL of cell suspension per well. Preparation of 20 µL of transfection complex: 5 µL of Opti-MEM and 5 µL of 10 nM dsRNA were mixed; 8.5 µL of Opti-MEM and 1.5 µL of RNAiMax transfection reagent were mixed and left to stand for 5 min; and then the above two mixtures were mixed and left to stand for 20 min. The transfection complex described above was added to a 24-well plate, and the plate was placed in a thermostatic incubator at 37 °C with 5% CO₂ for 48 h for successive culture. The cells were harvested 48 hours after transfection, and RNA extraction was performed using a TRIZOL method. A cDNA library was prepared using a TruSeq Stranded Total RNA Library Prep kit (Illumina), and sequencing was performed on the DNBSEQ platform. The original RNAseq reads were filtered using an SOAPnuke software from BGI genomics, and the specific steps are as follows: 1) removing reads containing a linker (linker contamination); 2) removing reads with unknown base N content greater than 5%; 3) removing low-quality reads (reads having a ratio of bases with quality values less than 15 to the total number of bases in the reads greater than 20% were defined as low-quality reads). Finally, the filtered reads were aligned with the human genome (GCF_000001405.39_GRCh38.p13) using HISAT (RNA-seq calibrator), and unique alignment reads were calculated by featureCounts. Differential gene expression analysis was performed by R package DESeq2. The results were as follows:

**Table 7: Results of off-target effect related to up-regulation and down-regulation of gene expression caused by dsRNAs**

| **siRNA sequence** | **Target** | **Control 1** | **AS strand modification site** | **Modified monomer** | **Number of down-regulated genes** | **Number of up-regulated genes** | **Total** | **Off-target effects** |
|---|---|---|---|---|---|---|---|---|
| E5 | **C5** | **VS blank** | / | / | **114** | **243** | **357** | / |
| FE5 | | **VS blank** | **Site 6** | **2M4Cm** | **10** | **57** | **67** | **-81.2%** |
| E17 | | **VS blank** | **/** | / | **76** | **130** | **206** | / |
| FE17 | | **VS blank** | **Site 8** | **2M4Cm** | **8** | **8** | **16** | **-92.2%** |
| E85 | | **VS blank** | **/** | / | **57** | **47** | **104** | |
| FE85 | | **VS blank** | **Site 8** | **2M4Cm** | **11** | **50** | **61** | **-41.3%** |

| | | | | | | | | |
|---|---|---|---|---|---|---|---|---|
| Note: In the above table, VS blank represents the change of genes by the target sequence relative to the blank control sequence (blank), in which UP represents the up-regulated genes; Down represents the down-regulated genes; and the sum of the two is the number of gene expressions possibly affected by the sequence in the statistical model; the higher the number, the more affected genes are represented, i.e., the greater the off-target possibility and extent are; the off-target effect is the reduced efficiency of the modified monomer groups relative to the unmodified groups. | | | | | | | | |

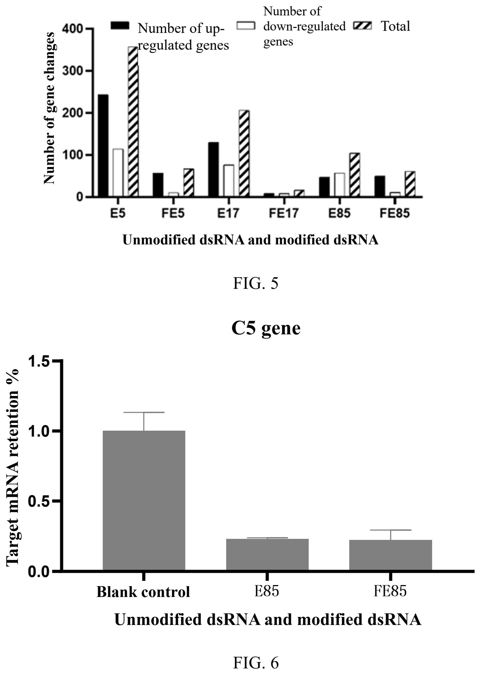

The experimental data further demonstrate that the incorporation of the modified cytidine of the present disclosure into sites 6-8 of the dsRNA AS strands was able to effectively reduce the off-target number of the target genes (results are shown in Table 7 and FIG. 5), indicating that the reduction in off-target effects by the modified cytidine of the present disclosure at sites 6-8 of the AS strands had predictable universality.

Furthermore, verification of the *in vitro* interference efficiency was performed taking E85 and FE85 as examples. Cell transfection followed the same transfection steps described above. Total cellular RNA was extracted from the cell sample according to the instructions of the RNA extraction kit and quantified using NanoDrop. The target genes and GAPDH gene in the total RNA of the cells were assayed by the duplex RT-qPCR assay. The relative expression level of the target genes in cells of other experimental groups was analyzed by a relatively quantified analytical method using a reference gene as a standard, wherein the GADPH gene was the reference gene, and the blank was the control. After the cells were transfected with the sequences described above, the relative activity of the target genes relative to the unmodified groups was assayed. The results are shown in Table 8 and FIG. 6:

**Table 8. Experimental results for effect of dsRNAs on knockdown activity**

| **siRNA sequence** | **AS strand modification site** | **Modified monomer** | **Change in knockdown activity relative to the unmodified groups** |
|---|---|---|---|
| E85 | / | / | / |
| FE85 | **Site 8** | 2M4Cm | +0.7% |

The results are shown in Table 8 and FIG. 6: the incorporation of the modified cytidine into sites 6-8 of the dsRNA AS strands effectively reduced the off-target number of the target genes while retaining a highly efficient inhibitory activity against the target genes. The inhibitory activities before and after the modification were comparable with no statistically significant difference (P < 0.05).

In summary, the incorporation of the modified nucleoside monomers provided by the present disclosure into sites 6-8 of the dsRNA AS strands was able to not only reduce the dsRNA off-target effects, but also maintain or even enhance to a certain extent the specific binding (expression suppression) effects on the target genes. Moreover, the effects described above repeated themselves in a variety of different sequences of different targets. Therefore, the modified nucleoside monomers of the present disclosure are expected to have development potentials for a variety of targets and sequences. Furthermore, the present disclosure reconfigured some native ribonucleoside monomers using simpler chemical synthesis steps and methods, and can also be expected to have lower physiological toxicity.

## Claims

1. A nucleoside monomer, having the structure of formula (I): 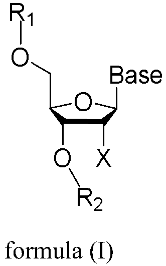 wherein, Base is selected from the following structures: 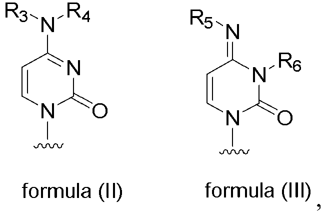
R₃, R₄, R₅, and R₆ are each independently selected from an amino protective group, hydrogen, C₁₋₃ alkyl (methyl, ethyl, propyl, and isopropyl), methoxyethyl, cyclopropyl, fluoromethyl, difluoromethyl, trifluoromethyl, and acetyl, provided that R₃ and R₄ are not both hydrogen, and R₅ and R₆ are not both hydrogen;
X is selected from F and -OMe;
R₁ and R₂ are each independently selected from H, a hydroxyl protecting group, and a phosphoramidite group.

2. The nucleoside monomer as claimed in claim 1, wherein R₃, R₄, R₅, and R₆ are each independently selected from an amino protective group, hydrogen, C₁₋₃ alkyl (methyl, ethyl, propyl, and isopropyl), and cyclopropyl.

3. The nucleoside monomer as claimed in claim 1, wherein Base is selected from

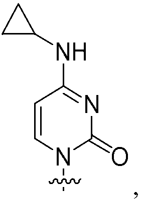

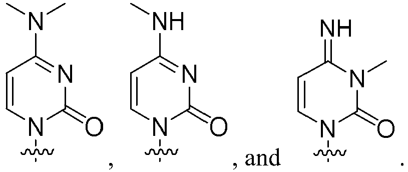

4. The nucleoside monomer as claimed in claim 1, wherein R₁ and R₂ are each independently selected from DMTr and a phosphoramidite group; preferably, R₁ and R₂ are each independently selected from DMTr and 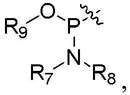 wherein R₇ and R₈ are each independently selected from C₁₋₃ linear or branched alkyl, and R₉ is selected from C₁₋₃ linear or branched alkyl and C₁₋₃ linear or branched alkyl substituted with cyano (-CN); preferably, R₁ and R₂ are each independently selected from DMTr and 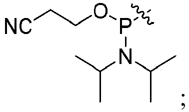 preferably, R₁ is selected from DMTr, and R₂ is selected from 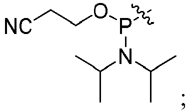 preferably, R₁ and R₂ are both selected from H.

5. The nucleoside monomer as claimed in claim 1, selected from:

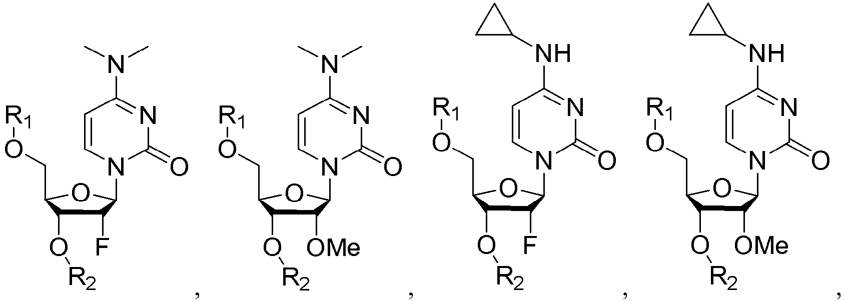

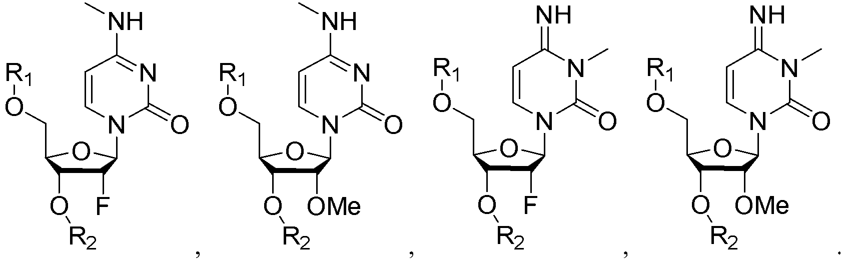

6. A double-stranded ribonucleic acid capable of inhibiting target gene expression, comprising a sense strand and an antisense strand, wherein each strand has 14-30 nucleotides, the sense strand optionally comprises a ligand, and at least one of positions 6, 7, or 8 from the 5' end of the antisense strand comprises a nucleoside monomer as shown below: 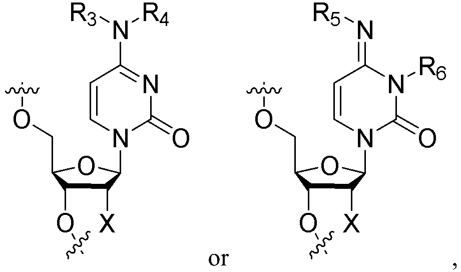 wherein R₃, R₄, R₅, and R₆ are each independently selected from hydrogen, C₁₋₃ alkyl (methyl, ethyl, propyl, and isopropyl), and cyclopropyl, provided that R₃ and R₄ are not both hydrogen, and R₅ and R₆ are not both hydrogen; X is selected from F and -Ome; the wavy line in the structure 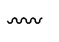 represents a site where a phosphate ester bond is formed.

7. The double-stranded ribonucleic acid as claimed in claim 6, wherein at least one of positions 6, 7, or 8 from the 5' end of the antisense strand comprises a nucleoside monomer as shown below: 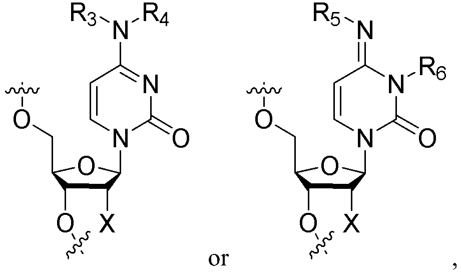 wherein R₃, R₄, R₅, and R₆ are each independently selected from hydrogen, methyl, and cyclopropyl, provided that R₃ and R₄ are not both hydrogen, and R₅ and R₆ are not both hydrogen.

8. The double-stranded ribonucleic acid as claimed in claim 6, wherein at least one of positions 6, 7, or 8 from the 5' end of the antisense strand comprises a nucleoside monomer as shown below:

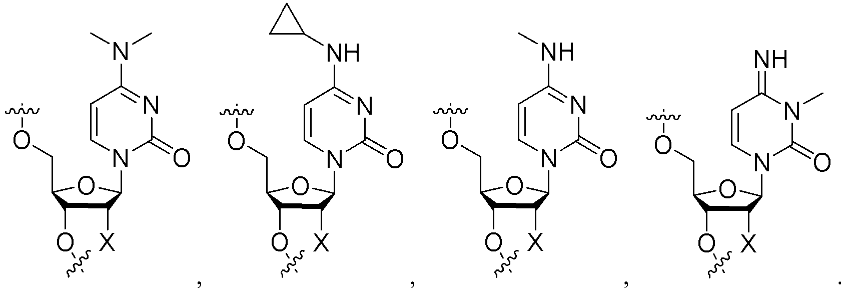

9. The double-stranded ribonucleic acid as claimed in any one of claims 6-8, wherein the sense strand comprises a ligand.

10. The double-stranded ribonucleic acid as claimed in claim 9, wherein the ligand is linked at the 5' end or the 3' end of the sense strand.

11. The double-stranded ribonucleic acid as claimed in claim 10, wherein the ligand is an asialoglycoprotein receptor (ASGPR) ligand.

12. The double-stranded ribonucleic acid as claimed in claim 11, wherein the ASGPR ligand is one or more GalNAc derivatives linked through a bivalent or trivalent branched structure.

13. The double-stranded ribonucleic acid as claimed in any one of claims 6-12, wherein the sense strand has 21 nucleotides and the antisense strand has 23 nucleotides.

14. A pharmaceutical composition, comprising the double-stranded ribonucleic acid as claimed in any one of claims 6-13 and a pharmaceutically acceptable carrier, excipient or delivery carrier.

15. A vector for inhibiting target gene expression in a cell, comprising a regulatory sequence operably linked to a nucleotide sequence encoding at least one strand of the double-stranded ribonucleic acid as claimed in any one of claims 6-13.

16. A cell, comprising the vector for inhibiting target gene expression in a cell as claimed in claim 15.

17. Use of the double-stranded ribonucleic acid as claimed in any one of claims 6-13 or the pharmaceutical composition as claimed in claim 14 or the vector as claimed in claim 15 for manufacturing a medicament for inhibiting or silencing target gene expression in a cell.

18. Use of the double-stranded ribonucleic acid as claimed in any one of claims 6-13 or the pharmaceutical composition as claimed in claim 14 or the vector as claimed in claim 15 for manufacturing a medicament for arresting off-target effects caused by the antisense strand of the double-stranded ribonucleic acid.

19. Use of the double-stranded ribonucleic acid as claimed in any one of claims 6-13 or the pharmaceutical composition as claimed in claim 14 or the vector as claimed in claim 15 for manufacturing a medicament for delivering the double-stranded ribonucleic acid to a specific target position in a subject.

20. The use as claimed in claim 19, wherein the target position is the liver.

21. The use as claimed in any one of claims 17-20, wherein the target gene is selected from: PCSK9, ANGPTL3, FXII, APOB, LPA, and C5.

22. A method for inhibiting target gene expression in a cell, comprising the following steps:
(a) introducing the double-stranded ribonucleic acid as claimed in any one of claims 6-13 into a cell, wherein the double-stranded ribonucleic acid can inhibit target gene expression by at least 40% when in contact with a cell expressing the target gene; and
(b) maintaining the cell obtained in step (a) for a sufficiently long time to degrade a mRNA transcript of the target gene, thereby inhibiting the expression of the target gene in the cell.

23. A method for treating and preventing a disease related to abnormal up-regulation of target gene expression, comprising administering to a patient in need of such treatment, prevention, or control a therapeutically effective amount or a prophylactically effective amount of one or more of the double-stranded ribonucleic acids as claimed in any one of claims 6-13 or the pharmaceutical composition as claimed in claim 14 or the vector as claimed in claim 15.
